# EUROPEAN PATENT SPECIFICATION

(11) **EP 2 477 585 B1**
(45) Date of publication and mention of the grant of the patent: **22.11.2017**
(21) Application number: 10760500.8
(22) Date of filing: 07.09.2010
(51) Int. Cl.: A61F 6/18, A61F 6/22

(54) **MINIMALLY INVASIVE DELIVERY DEVICES**
MINIMAL-INVASIVE FREISETZUNGSVORRICHTUNGEN
DISPOSITIFS D'ADMINISTRATION À EFFRACTION MINIMALE

(30) Priority: 17.09.2009 US 562057
(43) Date of publication of application: 25.07.2012
(73) Proprietor: Bayer Essure Inc., Whippany, NJ 07981 (US)
(72) Inventor: CRUZADA, Julian, Sunnyvale CA 94086 (US); STOUT, Christopher, San Bruno CA 94066 (US); SWANN, Betsy, Grass Valley CA 95945 (US)
(74) Representative: BIP Patents
(86) International application number: PCT/US2010/048025
(87) International publication number: WO 2011/034755

(56) References cited:
- WO-A2-2006/055692
- WO-A2-2006/105353
- US-A1- 2008 041 394
- US-B1- 6 709 667
- US-B1- 6 763 833

## Description

### FIELD

The present invention relates to the field of minimally invasive medical devices and procedures and, in particular, to devices and methods for transcervical gynecological procedures.

### BACKGROUND

Female contraception and/or sterilization may be affected by transcervically introducing an object (e.g. a coil) into a fallopian tube to inhibit conception. Devices, systems and methods for such a contraceptive approach have been described in various patents and patent applications assigned to the present assignee. For example, PCT Patent Application No. 99/15116, U.S. Pat. No. 6,526,979 and U.S. Pat. No. 6,634,361, describe devices that are transcervically inserted into an ostium of a fallopian tube and mechanically anchored within the fallopian tube. The devices described in these patents and patent application may promote a tissue in-growth around and within the inserted device, which may be referred to as an implant or an insert. One example of such devices is the device known as "Essure" from Conceptus, Inc. of Mountain View, Calif. This tissue in-growth tends to provide long-term contraception and/or permanent sterilization without the need for surgical procedures.

U.S. Patent No. 6,709,667 and U.S. Publication No. 2008/0041394, which are assigned to the present assignee, describe delivery systems and methods for these devices. The delivery system is typically formed of a handle, a delivery catheter system, and a guidewire onto which is held the contraceptive implant to be placed within the fallopian tube. The delivery catheter system contains the guidewire, a release catheter and the contraceptive implant and the guidewire within the release catheter.

The delivery catheter system is transcervically positioned into the uterus and the fallopian tubes via a hysteroscope. Example of hysteroscopes are described in U.S. published applications 2006/0293560 and 2005/0288551. The delivery catheter system and guidewire enter the hysteroscope through a working channel of the hysteroscope. A distention valve is typically positioned at the tip (proximal end) of the working channel. The distention valve seals the entrance of the working channel to prevent a distention fluid, such as saline, to flow out of the hysteroscope as a device, such as the delivery catheter system and guidewire of the intrafallopian contraceptive delivery device, is introduced into the working channel. The opening into the distention valve is designed to prevent the leakage of any fluid out of the hysteroscope and therefore has the smallest opening possible to allow a very tight fit between the device and the valve opening. To prevent damaging the tip of the guidewire or the contraceptive implant to be inserted into the fallopian tube, the guidewire and delivery catheter system are introduced into the distention valve through an introducer sheath. Some introducer sheaths are formed of a soft flexible material such as plastic or Teflon and have a slit to aid in grasping and in the removal of the introducer sheath after the delivery system has passed through the distention valve. These flexible introducer sheaths must therefore be inserted into the opening of the distention valve while on a stiff mandrel. Once the mandrel is placed within the distention valve and the channel to the desired depth the mandrel is removed, leaving the introducer sheath within the working channel and the distention valve. After placing the introducer sheath into the distention valve and removing the mandrel, the tip of the guidewire and the delivery catheter system may be inserted through the distention valve using the introducer sheath and into the working channel. The introducer sheath may then be removed or may be kept in place throughout the procedure. The distention valve may have a tight opening that places pressure on the delivery catheter and causes friction when withdrawing the delivery catheter. The distention valve prevents fluid leakage from the working channel. When the introducer sheath is inserted through the distention valve, fluid can spray out of the valve and onto the physician or physician's assistant. The amount of fluid spray-back can be significant depending on the fluid pressure during the procedure. In addition, friction between the introducer sheath and distention valve can be problematic.

Once a physician has positioned the delivery catheter system and the guidewire at a position within the fallopian tube where the contraceptive implant may be deposited, it may be awkward and difficult for the physician to maintain the position of the delivery system relative to the anatomy and may require the physician to use an assistant to aid in the proper stabilization of the system relative to the hysteroscope. In addition, some of the contraceptive implant devices in the above references require disengaging from a delivery catheter by using an axial torque. In practice this requires the delivery catheter to be fully rotated multiple times to disengage a contraceptive implant device from the delivery system in order to deposit the contraceptive implant device into a fallopian tube. This maneuver may be difficult and cumbersome to perform considering that the device must also remain axially aligned in the fallopian tube.

A stabilization device may be used to help with this awkwardness. One such stabilization device includes a handle that includes a contoured holster to couple the hysteroscope and the handle of the control device, creating a fixed distance between the hysteroscope and the control device. The stabilization device may also be coupled to an endoscope. Examples of such stabilization devices are described in U.S. published application 2006/0293560 and U.S. published application 2008/0041394, Document US2008/0041394A1 discloses a system for delivering an implant.

Once the delivery catheter system is positioned at the proper position in the fallopian tube, the implant is exposed, and realigned if necessary, in the fallopian tube, expanded, and released from the delivery catheter system by rotating a thumbwheel on the handle. One problem with this thumbwheel is that the wheel sometimes sticks causing the doctor to look away from the hysteroscope.

### SUMMARY OF THE DESCRIPTION

Various different embodiments are disclosed below and the following summary provides a brief description of only some of these embodiments. According to one aspect of the invention, certain embodiments described below relate to a medical device that provides a transcervical pathway and also stabilizes a device for a minimally invasive gynecological procedure. The device for the minimally invasive gynecological procedure may be an intrafallopian contraceptive delivery device.

One embodiment of the present invention relates to a system for delivering an implant to an ovarian pathway (e.g., a fallopian tube) of a female body that includes a delivery sheath having a first longitudinal opening and a second longitudinal opening wherein the first longitudinal opening is configured to receive a visualization system; and an implant delivery device having an implant sheath positionable within the second longitudinal opening and a delivery catheter (which may be a delivery wire) deliverable through the implant sheath, wherein the implant delivery device is lockable to the delivery sheath and wherein the delivery catheter is movable longitudinally and rotationally relative to the delivery sheath when the implant delivery device is locked to the delivery sheath.

Another embodiment of the present invention relates to a system for delivering an implant to an ovarian pathway of a female body including a delivery sheath having a first longitudinal opening and a second longitudinal opening; a visualization system deliverable through the first longitudinal opening; and an implant delivery device having a protective sheath and an implant sheath positionable within the second longitudinal opening and a delivery catheter deliverable through the protective sheath, wherein the delivery catheter and the implant sheath are more flexible than the protective sheath.

In a further embodiment, the present invention relates to an implant delivery system having a sheath having a first end and a second end; a first longitudinal opening in the sheath having a port at the first end of the sheath to receive an implant delivery system, the port having an interlocking element to lock the implant delivery system to the sheath wherein the implant delivery system is lockable to the sheath while still retaining the ability to move a deliverable implant longitudinally relative to the sheath when the sheath is stationary; and a second longitudinal opening in the sheath to receive an implant viewing system.

In yet another embodiment, the present invention relates to a system for delivering an implant to an ovarian pathway of a female body comprising: a guide; a protective sheath extending from the guide; a handle moveably (e.g. slideably) engaged with the guide; a release catheter having a proximal end and a distal end, the proximal end of the release catheter connected to the handle, the release catheter slideable through the protective sheath; and an implant releasably coupled with the distal end of the release catheter. The implant may comprise an expandable tubular member and a tissue in-growth agent, and the implant can be moved longitudinally, relative to the guide, by moving the handle along the guide.

In yet another embodiment, the present invention relates to a method of using a fallopian tube implant. This method may include: attaching the fallopian tube implant to a hysteroscope sheath; attaching a hysteroscope to the hysteroscope sheath; and moving a handle which is moveably coupled to a guide that is couple to the hysteroscope. As the handle is moved longitudinally, an implant, couple to a distal end of a release catheter, is also moved longitudinally. A proximal end of the release catheter is coupled to the handle. As the handle is moved rotationally, while the guide is locked to the hysteroscope, the implant is also moved rotationally without having to rotate the hysteroscope sheath; this can be useful in general and in particular for implant devices which have a premanufactured, built-in, bend or conformation (e.g., a 15° angular bend) as certain implant devices in the prior art.

In yet another embodiment, a system for delivering an implant to a fallopian tube includes a port, a handle coupled to the port and a control which is moveably coupled to the handle and which is configured, when the control is moved along on the handle, to move longitudinally the implant. The port is configured to be rotatably coupled to a working channel of a hysteroscope assembly. The implant is coupled to a release catheter which is coupled to the control, and the implant is moved, relative to the handle by the control. The implant may be moved longitudinally relative to the handle by moving the control along the handle, and the implant may be rotated, relative to a stationary hysteroscope assembly, by rotating the handle. The handle can be moved rotationally without releasing a valve on the working channel and the control (and hence the implant) can be moved without releasing a valve on the working channel. The port can include a ball configured to rotatably couple to a socket configured to receive the ball; the socket can be part of the working channel or an adapter designed to attach to the working channel. The ball and the socket each include a lumen designed to allow passage of the implant sheath and the release catheter. In one embodiment, the hysteroscope assembly is a hysteroscope sheath which is designed to receive a hysteroscope (through an imaging channel) and the port (through a working channel); in another embodiment the hysteroscope assembly is a hysteroscope with an integrated working channel. In one embodiment, a lumen of a protective sheath can be coupled to the lumen of the port and can provide protection to the implant sheath and release catheter which extend through a lumen of the protective sheath.

Various other devices and methods for using devices, including kits for use in treating patients, are also described below. Other features of the present invention will be apparent from the accompanying drawings and from the detailed description that follows.

### BRIEF DESCRIPTION OF THE DRAWINGS

The invention is described by way of example with reference to the accompanying drawings, wherein:
Figure 1 is a schematic view illustrating the uterine and tubal anatomy for deployment of the contraceptive devices;
Figure 1A is a flow diagram of a method for delivering a contraceptive device in accordance with one embodiment of the invention;
Figure 2 is a front perspective view of a delivery sheath in accordance with one embodiment of the invention;
Figure 3 is a rear perspective view of the delivery sheath of Figure 2 in accordance with one embodiment of the invention;
Figure 4 is a perspective view of the delivery sheath, guide, device delivery system and hysteroscope in accordance with one embodiment of the invention; Figure 4A is a cross sectional view of the distal portion of the delivery sheath of Figure 4; Figure 4B is a cross sectional view of the delivery system shown in Figure 4;
Figure 5 is a side view of the guide and device delivery system in accordance with one embodiment of the invention;
Figure 6 is a top view of the guide in accordance with one embodiment of the invention;
Figure 7A is a side sectional view of a handle of the device delivery system in accordance with one embodiment of the invention;
Figures 7B - 7D illustrate actuation of actuators on the handle of Figure 7A in accordance with one embodiment of the invention;
Figure 7E is a side sectional view of a handle of the delivery device system in accordance with one embodiment of the invention;
Figures 7F-7H illustrate actuation of actuators on the handle of Figure 7E in accordance with one embodiment of the invention;
Figures 8A and 8B are side views of a distal end of the device delivery system in accordance with one embodiment of the invention;
Figure 9 is a side view of the implant in accordance with one embodiment of the invention;
Figure 10 is a schematic view illustrating delivery of the implant to a fallopian tube with the delivery sheath in accordance with one embodiment of the invention;
Figures 11A and 11B are schematics view illustrating lateral movement of the handle relative to the guide;
Figure 11C is a schematic view illustrating rotational movement of the handle relative to the guide;
Figure 12 is a schematic view illustrating actuation of a first button on the handle to deliver the implant to the fallopian tube in accordance with one embodiment of the invention;
Figure 13 is a schematic view illustrating actuation of a second button on the handle to deliver the implant to the fallopian tube in accordance with one embodiment of the invention;
Figures 14A and 14B illustrate an alternative embodiment of the handle in accordance with one embodiment of the invention;
Figure 15 is a perspective view of an alternative embodiment of the handle in accordance with one embodiment of the invention;
Figure 16 is a perspective view of the handle of Figure 15 with the delivery sheath and hysteroscope in accordance with one embodiment of the invention;
Figure 17 is a perspective view illustrating connection of the handle of Figure 15 with the delivery sheath in accordance with one embodiment of the invention;
Figure 18 is a perspective view illustrating delivery of the delivery catheter through the delivery sheath in accordance with one embodiment of the invention;
Figures 19 and 19A illustrate rotational movement of the delivery catheter with the handle in accordance with one embodiment of the invention;
Figure 20 is a perspective view of another embodiment of a handle in accordance with one embodiment of the invention;
Figure 21 is a perspective view of the handle of Figure 20 with the delivery sheath and hysteroscope in accordance with one embodiment of the invention; and
Figures 22 and 23 are perspective views illustrating a steerable handle in accordance with one embodiment of the invention.
Figure 24A shows a perspective view of another embodiment of a delivery device, shown in this view without a protective sheath;
Figure 24B shows a perspective view of the delivery device of Figure 24A with a protective sheath attached to the delivery device;
Figure 24C shows an example of a conventional hysteroscope and hysteroscope sheath with a working channel which can receive the protective sheath and the delivery device;
Figure 25A shows a cross sectional view of the protective sheath and a distal portion of the delivery device;
Figure 25B shows a cross sectional view of the hysteroscope sheath;
Figure 25C shows a cross sectional view of the hysteroscope sheath with the delivery device and the protective sheath inserted into the working channel of the hysteroscope sheath;
Figure 26 shows a cross sectional view of the delivery device;
Figure 27 shows a view down the working channel of the hysteroscope sheath.

### DETAILED DESCRIPTION

Embodiments of the present invention relate to self-aligning, easy locking and/or one-handed systems and methods for delivering a contraceptive device to an ovarian pathway of a patient. For example, the system may include a delivery sheath having a first longitudinal opening and a second longitudinal opening. A visualization system, such as a hysteroscope, is deliverable through the first longitudinal opening of the delivery sheath. The delivery sheath may also be referred to as a hysteroscope sheath. An implant delivery system is deliverable through the second longitudinal opening of the delivery sheath. In one embodiment, the implant delivery system includes a guide having a body, a rail extending proximally from the body, and a protective sheath extending distally from the body. The guide is locked to the delivery sheath at the second longitudinal opening of the delivery sheath, and the protective sheath extends through the second longitudinal opening of the delivery sheath. The implant delivery system may also include a handle, an implant sheath, a release catheter, and an implant releasably coupled with the release catheter. The release catheter may also be referred to as a deliver catheter. The implant sheath and release catheter each have a proximal end and a distal end, and the proximal ends of the implant sheath and the release catheter are connected to the handle. The implant sheath, the implant, and the release catheter are slideable through the protective sheath which is configured to be disposed within the delivery sheath. The handle of the implant delivery system is slideably engaged with the rail of the guide to position the implant in the ovarian pathway. Actuators on the handle of the implant delivery system can retract the implant sheath, expand the implant, and release the implant from the release catheter.

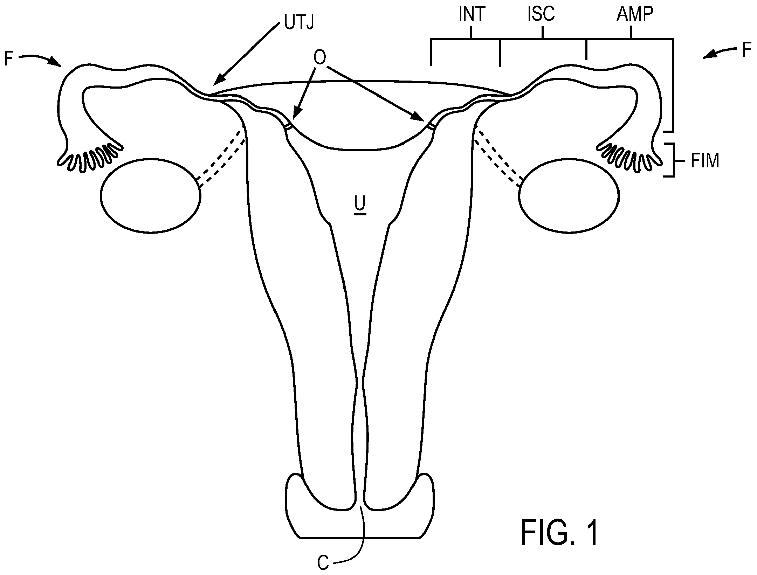

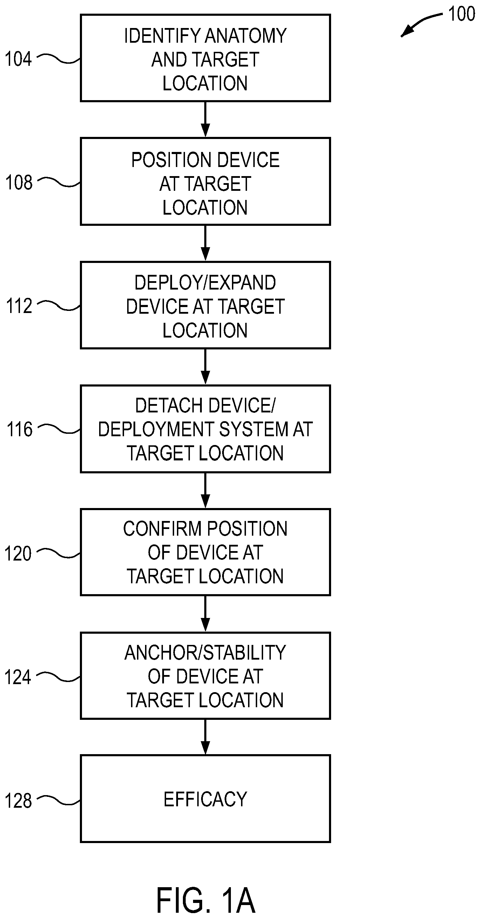

Referring now to Figure 1, access to uterus U is gained through the cervix C. From within the uterus U, the fallopian tubes F are accessed via the tubal ostiums O. The fallopian tubes F generally include three segments between the ostium O and the fimbria FIM. Beginning adjacent the uterus U, the intramural segment INT of the fallopian tubes F are surrounded by the muscular uterine tissues. Beginning at the uterotubal junction UTJ, the fallopian tubes F extend beyond the uterine tissues and within the peritoneal cavity along an isthmic segment ISC, and then along an ampullary segment AMP.

Referring now to Figure 1A, an exemplary method 100 for delivering a contraceptive device to a fallopian tube is illustrated. It will be appreciated that the exemplary method 100 may include fewer or greater number of operations than those described below. In addition, the operations may occur in different orders than described or may occur simultaneously.

The method 100 begins at block 104 by identifying the anatomy and target location. The operator determines a preferred placement of the contraceptive device within the ostium, and also determines if any special circumstances are present for a particular device placement procedure. Anatomy and target location identification can be facilitated using a variety of known visualization modes, including hysteroscopy, sonography (ultrasound), fluoroscopy, and the like.

The method 100 continues at block 108 by positioning the device at the target location. A wide variety of techniques may be used to assist a healthcare professional in positioning the device in the correct location, including visualization techniques, providing high-contrast markers (such as radiopaque markers, echogenic markers, or the like), providing tactile indication of the placement position by including physical stops or "bumpers" (which may be adapted to engage reference tissues in such a tactile way as to send a signal to the healthcare professional), or the like.

The method 100 continues by deploying and/or expanding the device at the target location (block 112), and detaching the device from the deployment system (block 116). The method 100 may continue by confirming the position of the device at the target location (block 120). Confirmation may be provided by visualizing at least a portion of the device after detachment, often using the same visualization modality used during placement. In addition to optical visualization techniques, confirmation of delivery may be provided by including radiopaque markers for fluoroscopic placement confirmation, sonographic markers for ultrasound placement confirmation, or the like.

The method 100 may include anchoring and stabilizing the device at the target location (block 124) and verifying the efficacy (block 128) of the deployed device. Efficacy may be provided by incorporating a lumen/space filling design in the implant, such as polyester fibers to incite a tissue reaction (i.e., tissue in-growth fibers), that sufficiently alters the function and architecture of the fallopian tube so as to inhibit conception. This tissue reaction results in the incorporation of the contraceptive device into the tubal lumen tissues, so that the device is firmly embedded into the surrounding tissue structure.

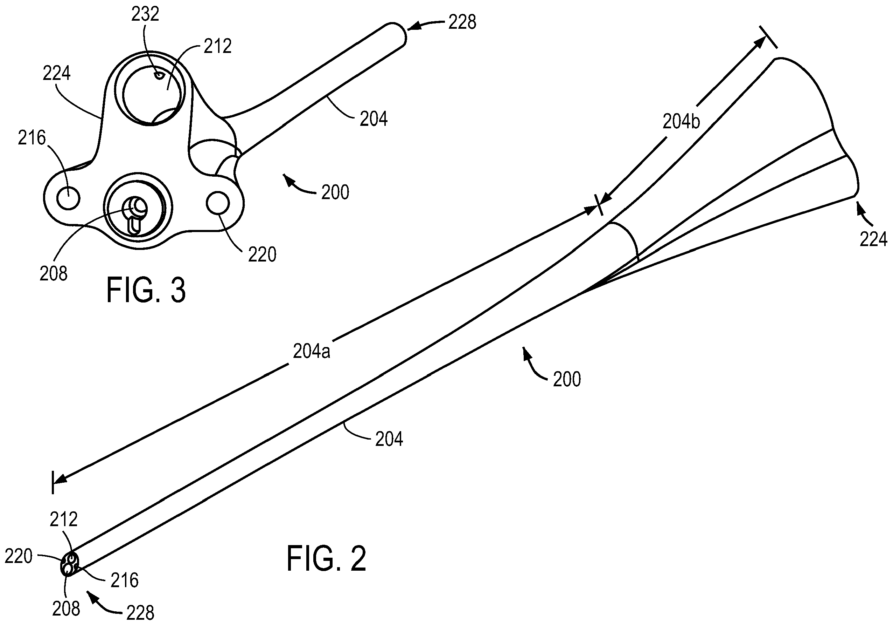

Figures 2 and 3 illustrate a delivery sheath 200. This delivery sheath may also be referred to as a hysteroscope sheath. The delivery sheath 200 is configured to deliver a contraceptive device to a fallopian tube through the cervix C and uterus U. The delivery sheath 200 includes an elongate body 204 that includes a first lumen 208 and a second lumen 212. The elongate body 204 may optionally include a third lumen 216 (e.g. a fluid inlet lumen) and/or fourth lumen 220 (e.g., a fluid outlet lumen).

The elongate body 204 includes a proximal end 224 and a distal end 228. As shown in Figures 2 and 3, the proximal end 224 of the elongate body 204 is larger than the distal end 228 of the elongate body 204. The proximal end 224 extends out of the cervix and the patient such that an operator can deliver the contraceptive device through the sheath 200, while the distal end 228 extends to or near the ostium O.

The length of the elongate body 204 is configured to provide a transcervical pathway. For example, the length of the elongate body may be about 29-30 cm. In one embodiment, the elongate body 204 includes a working length 204a that extends through the cervix C and uterus U to or near the ostium O, and a manipulating length 204b that extends away from the cervix C and out of the female patient. In one embodiment, the working length 204a may be about 19-20cm. It will be appreciated that the length of the elongate body may be less than 29cm or greater than 30cm and the working length may be less than 19cm or greater than 20cm.

The first lumen 208 extends from the proximal end 224 to the distal end 228 of the elongate body. The first lumen 208 is configured to receive a visualization tool, such as, for example, a hysteroscope. The visualization tool may be secured to the sheath 200 through a locking mechanism, such as, for example, a groove and tab, at a proximal end of the first lumen 208. Alternatively, the visualization tool is not secured to the sheath 200.

The hysteroscope is positioned in the delivery sheath so that an operator can view the fallopian tube sufficiently to deliver the implant in the fallopian tube. In one embodiment, the distal end of the hysteroscope is aligned with the distal end of the sheath 200 (i.e., the distal end of the hysteroscope is flush with the distal end of the sheath 200). In another embodiment, the hysteroscope extends just beyond the distal end 228 of the elongate body 204. For example, the hysteroscope may extend less than about 1cm beyond the distal end 228 of the elongate body.

The second lumen 212 also extends from the proximal end 224 to the distal end 228 of the elongate body. The second lumen 212 is configured to receive a device delivery system. The device delivery system may be secured to the sheath 200 through a locking mechanism, such as, for example, a groove and tab at a proximal end of the second lumen 212. As shown in Figure 3, the second lumen 212 includes a tab 232 to interlock the delivery sheath with a corresponding groove in a device delivery system, described in further detail hereinafter.

The first lumen 208, second lumen 212, both the first lumen 208 and second lumen 212 or neither the first lumen 208 nor the second lumen 212 may include a distension valve to prevent fluid leakage through the sheath 200.

The optional third lumen 216 and fourth lumen 220 are configured to be fluid lumens. For example, the third lumen 216 may be a fluid inlet and the fourth lumen 220 may be a fluid outlet, or vice versa. Alternatively, a single lumen (e.g., only third lumen 216) is provided. When a single lumen is provided, the single lumen may be both a fluid inlet and fluid outlet, or the single lumen may be a fluid inlet, while the second lumen 212, for example, serves as the fluid outlet as well as a working channel.

In one embodiment, one or more tendons are connected to the distal end of the sheath 200 to allow the operator to deflect the sheath 200 (through controls at the proximal end which are couple to the tendons) to direct the distal end of the sheath 200 toward the ostium O. The sheath 200 and the hysteroscope 400 may, in one embodiment, have distal ends which are flexible.

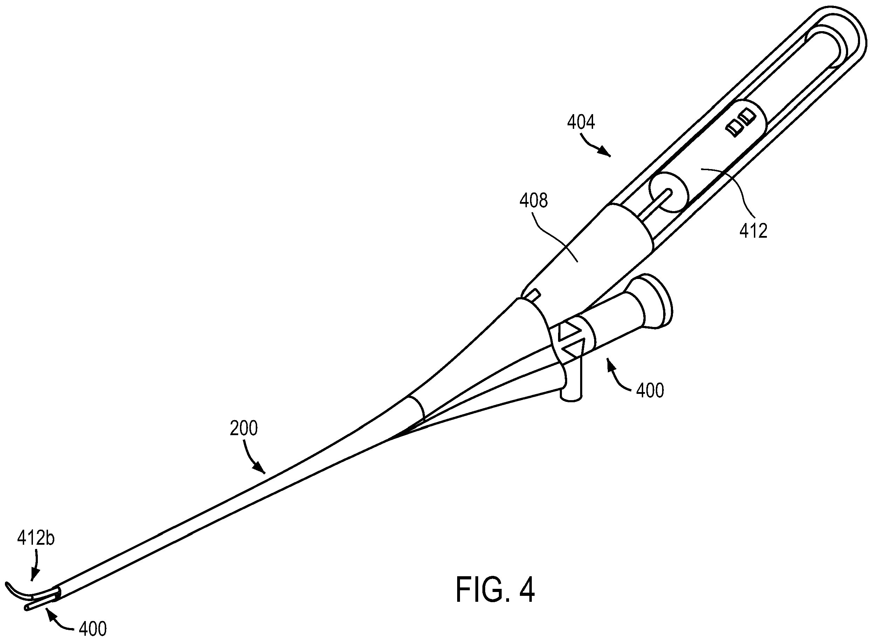

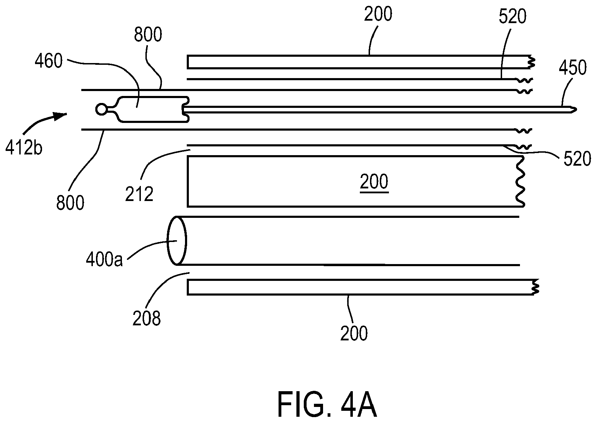

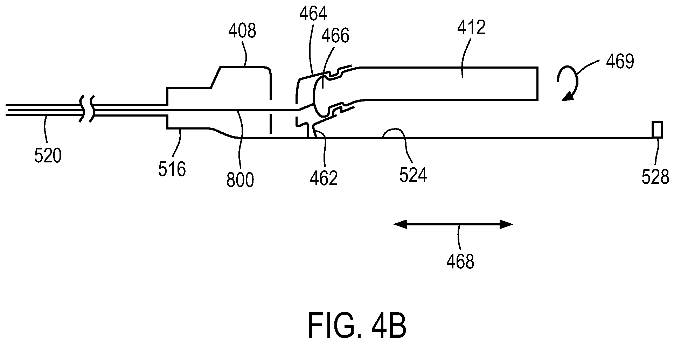

Figure 4 illustrates the sheath 200 with a hysteroscope 400 positioned in the first lumen 208 of the sheath 200 and a device delivery system 404 positioned in the second lumen 212 of the sheath 200. The delivery system 404 includes a guide 408 and a delivery device 412. As shown in Figure 4, the proximal end of the delivery device 412 extends from the proximal end of the delivery sheath 200 and the distal end 412b of the delivery device extends from the distal end of the delivery sheath 200. The distal end of the delivery device 412b includes the implant to be delivered to the ovarian pathway of the patient, and the proximal end 412 is configured to allow an operator to manipulate the delivery device to position and deliver the implant in the proper location in the ovarian pathway. This proper location is known in the art. The delivery sheath 200 allows for movement of the hysteroscope 400 or delivery device 412 independent of one another. The guide 408 is secured to the sheath 200, and the delivery device 412 is moveably (e.g. slideably) coupled to the guide 408.

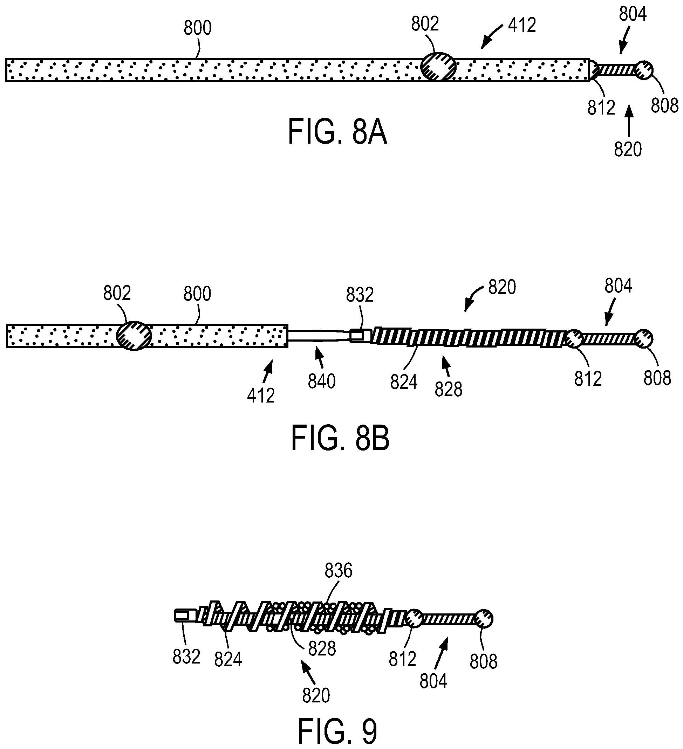

Figure 4A shows an enlarged cross sectional view of the distal end of the delivery sheath 200 of Figure 4. This enlarged view shows the hysteroscope 400's distal end 400a extending through and slightly out of the lumen 208 of the delivery sheath 200. This distal end 400a conveys an image through the hysteroscope 400 to the proximal end of the hysteroscope 400. The distal end 412b of the delivery device 412 is shown extending through and slightly out of the lumen 212 of the delivery sheath 200. The distal end 412b of the delivery device includes a protective sheath 520, which surrounds and protects the implant sheath 800, a release catheter 450, and an implant (or other device) 460. The implant 460 may be the contractive device 820 shown in Figures 8A, 8B and 9, and the release catheter 450 may be the same as release catheter 840 in Figure 8B; this device 820 is designed to be deployed within a fallopian tube and, after deployment it expands or enlarges to resilently engage the walls of the fallopian tube. This device 820 may include agents, such as polyester or Dacon fibers, which are designed to cause tissue-in-growth into the device. After time (e.g. several weeks), the tissue-in-growth into and around the device will effectively and functionally occlude the fallopian tube as is known in the art. The implant sheath may, in certain embodiments, be configured to cover and protect the implant 460 as it is being positioned with the fallopian tube but before it is deployed. In certain embodiments, the distal end of the implant 460 may be protected by the implant sheath 800 (as shown in Fig 4A) while the implant 460 is being positioned in a fallopian tube; in other embodiments, the distal end of the implant 460 may be used as, in effect, a guidewire, and may include an atramatic distal tip which extends beyond the implant sheath 800 (while the rest of the implant 460 remains covered by the implant sheath 800). The implant sheath 800 may radially restrain the implant 460 before the implant is deployed (e.g. released from the implant sheath), and after the implant sheath 800 releases the implant, the implant 460 can expand to resilently engage the walls of the fallopian tube. The release catheter 450 (which is also referred to as a delivery catheter) is, in one embodiment, releasably coupled to the implant 460; the implant 460 may be coupled to the release catheter 450 by a variety of ways known in the art, including a friction fit or a screw connection. The release catheter 450 may serve to hold the implant 460 at a position while the implant sheath 800 is retracted or may serve to push the implant 460 out of the implant sheath (which may be stationary or may also move). In one embodiment, the release catheter 450 may be a delivery wire which is coupled to the implant 460 by a frictional fit between a distal portion of the delivery wire and a proximal portion of the implant 460; in one implementation of this embodiment, the distal portion of the delivery wire is coupled, by a frictional fit, to an inner coil of an implant device which also includes an outer tubular structure such as an outer coil. The release catheter 450 in one embodiment is coupled to a control near the proximal end of the delivery device 412, and this control is configured to, when actuated, cause the release of the implant 460. The implant sheath 800 in one embodiment is coupled to a control (which can be an additional control which is separate from the control for the release catheter) near the proximal end of the delivery 412, and this control is configured to retract the implant sheath 800, to thereby expose the implant 460.

Figure 4B is a cross sectional view of one embodiment of the delivery system 404 of Figure 4. The delivery device 412 is coupled to the rail 524 of the guide 408 by a tab 462 which rides in the rail 524 in order to allow longitudinal movement 468 of the delivery 412 (which acts as a handle) to in turn longitudinally move the release catheter 450 and the implant sheath 800. The rail 524 may be a groove in the guide 408, and the tab 462 is designed to slidably fit within the groove to allow the delivery device 412 to be moved longitudinally (in a proximal or distal direction) as shown by the arrow 468. The tab 462 is coupled to a socket joint 464 which is designed to receive and hold a ball 466 near the distal end of delivery device 412. The ball 466 has a lumen (not shown) to allow the implant sheath 800 and the release catheter 450 within the implant sheath 800 to pass through the ball and be connected with controls, such as buttons, on the delivery device 412 so that the implant sheath 800 can be retraced and the implant 460 can be released. The socket joint 464, the interlock 516 and the guide 408 also include a lumen, which is in fluid communication with the lumen in the protective sheath 520, and these lumens are configured to allow passage of the implant sheath 800 and the release catheter 450. The ball 466 and the socket 464 are rotatably coupled so that the delivery device 412 can be rotated, as shown by arrow 469, while it is coupled to the socket 464. This allows the operator to rotate the implant 460, coupled to the release catheter 450, by rotating the delivery device 412; the operator can rotate the delivery device 412 (or move it longitudinally) without moving the delivery sheath and without moving the hysteroscope. The ability to rotate the implant 460 in this manner can be useful, particularly if the implant 460 has a preformed bend or shape which may require rotation of the implant in order to place in the implant in a proper position within a fallopian tube.

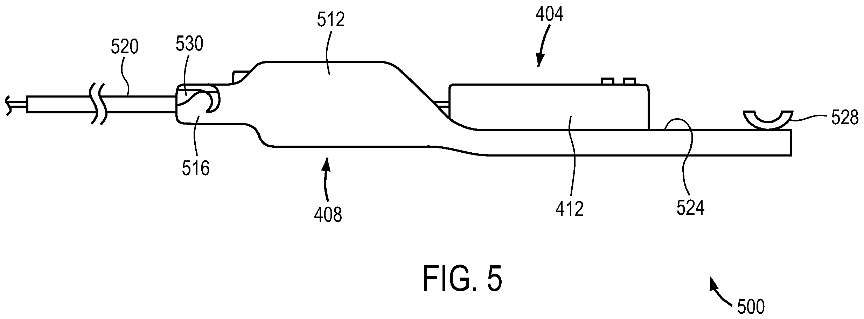

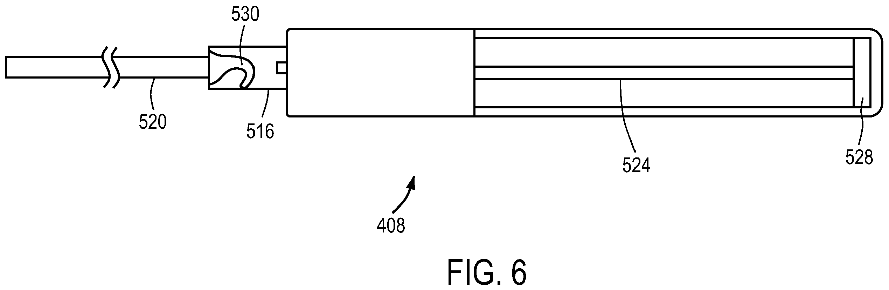

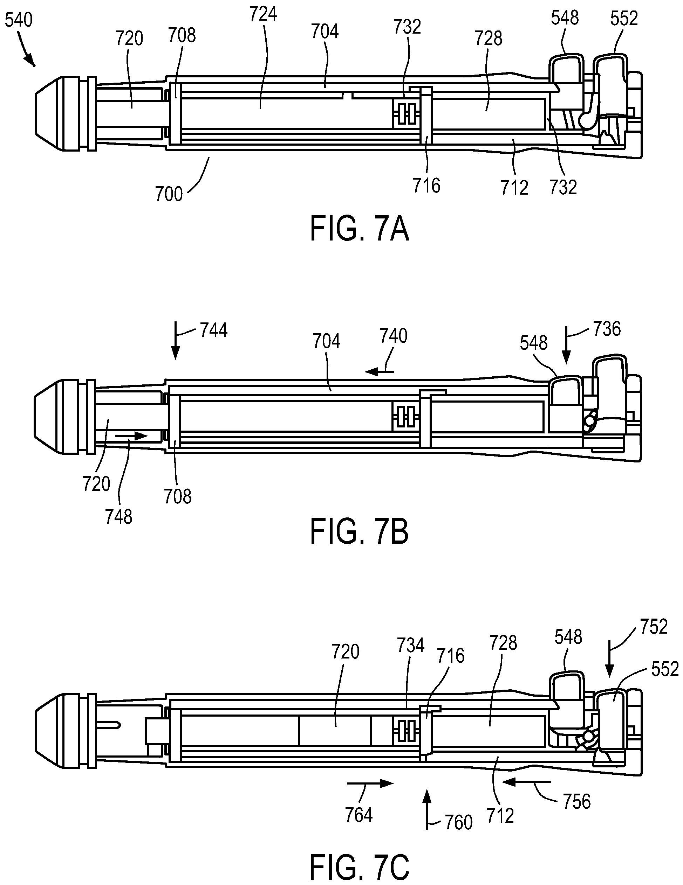

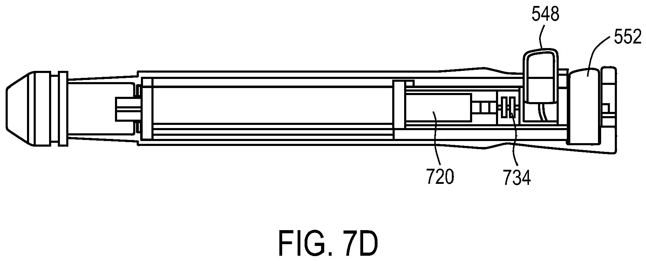

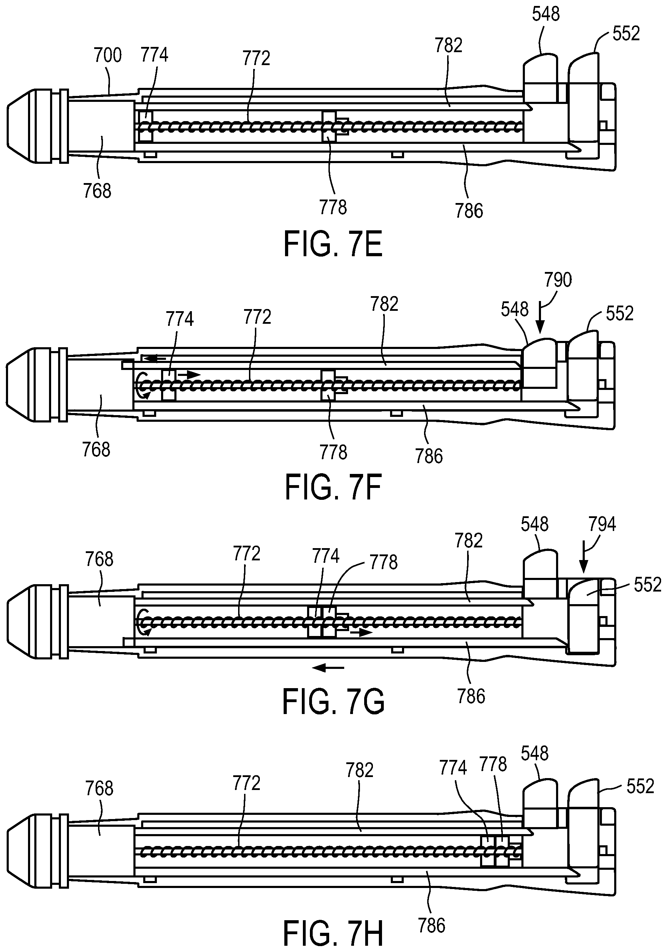

Figure 5 illustrates the guide 408 and delivery device 412 in further detail. Figure 6 illustrates the guide 408 in further detail, and Figures 7-9 illustrate one embodiment of the delivery device 412 in further detail. As described above, the guide 408 is secured to the sheath 200, which allows an operator to manipulate the delivery device 412 with one hand. The guide 408 also helps protect the delivery device 412 from damage.

The delivery device 412 is coupled to the guide 408, such that the handle of the delivery device can slide longitudinally relative to the delivery sheath 200. In one embodiment, the delivery device 412 is preloaded in the guide 408. In another embodiment, the delivery device 412 is loaded through the guide 408 by an operator.

Referring to Figure 5 in combination with Figure 6, the guide 408 includes a body 512, an interlock 516, a protective sheath 520 (which may be made of rigid metal), a rail 524 and a stopper 528.

The interlock 516 has a diameter that is smaller than the body 512 and is configured to be inserted into the second lumen 212 of the sheath 200. The shape and size of the interlock 516 is complementary to the shape and size of the second lumen 212 of the sheath. The interlock 516 may also include a groove 530. The groove 530 is configured to engage with the tab 232 in the second lumen 212 to lock the guide 408 to the sheath 200.

The protective sheath 520 is, in one embodiment, hard and is configured to protect the outer sheath (also referred to as the implant sheath) of the delivery device 412 from damage. The sheath 520 is configured to be inserted into the second lumen 212 at the proximal end and may extend to the distal end 228 of the sheath 200. In one embodiment, the distal end of the protective sheath 520 is aligned with the distal end 228 of the sheath 200 (i.e., the distal end of the protective sheath 520 is flush with the distal end 288 of the delivery sheath 200). Alternatively, a portion of the protective sheath 520 may extend beyond the distal end 228 of the sheath 200. The protective sheath 520 may be made of a metal which is both protective and bendable.

An exemplary length of the sheath 520 is about 29-30cm. It will be appreciated that the length of the sheath 520 may be less than 29cm or greater than 30cm. In one embodiment, the sheath 520 includes a distension valve to prevent fluid leakage through the sheath 520.

An exemplary material for the sheath 520 is stainless steel. It will be appreciated that the sheath 520 can be formed from other materials, such as, for example, other metals, hard plastics or composites.

The rail 524 is configured to allow the handle of the delivery device 412 to slide along the rail 524. Thus, the rail 524 is a slider assembly. The shape of the rail 524 can be generally complementary to the shape of at least a portion of the handle of the delivery device 412. For example, if the handle is curved, the rail 524 may be also be curved, the diameter of the curve of the rail 524 being slightly larger than the diameter of the handle.

The rail 524 extends distally away from the body 512. The stopper 528 is provided at a distal end of the rail 524 to limit movement of the handle relative to the body 512. In one embodiment, the length of the rail 524 is any value or range of values between about 1 and 10cm in length to allow for about 1-10cm of corresponding movement of the handle. Thus, the rail 524 both allows longitudinal movement of the delivery device 412 (and hence the implant) without requiring longitudinal movement of the hysteroscope but also limits the longitudinal movement of the delivery device 412.

The rail 524, in one embodiment, is sufficiently deep to allow for movement of the handle without the handle coming off the rail 524, but sufficiently shallow to allow for removal of the handle from the rail 524. The rail 524 may have openings (not shown) to allow an operator to more easily remove the handle from the rail 524. In addition, the rail 524 may have a track (not shown) to improve movement along the rail 524.

The guide may allow, in certain embodiments, for rotational movement of the rail relative to the delivery sheath 200. For example, the body of the guide may include a ball and socket joint (not shown) which is designed to receive a ball attached to the handle of the delivery device 412.

The handle of the delivery device 412 positions the implant at a correct location in the fallopian tube by sliding the handle of the delivery device 412 on the rail 524 which in turn moves the release catheter 450. The handle and/or rail 524 may optionally allow for rotational movement of the implant relative to the sheath, as described herein. The handle can include at least one control for deploying the implant. In the example shown in Figure 7A, the handle 540 includes two controls, actuator 548 and actuator 552.

Actuation of the first actuator 548 expands the contraceptive device and, actuation of the second actuation 552 releases the contraceptive device from the delivery device 412. In one embodiment, first actuator 548 and second actuator 552 are both push buttons; alternatively, one or the other of the first actuator 548 or second actuator 552 is a push button. Other exemplary actuators that can be used for one or both of the actuators 548, 552 include a toggle switch, a trigger, a tab slider, a thumb wheel and the like. In one embodiment, the handle 540 includes an auxiliary actuator, such as a thumb wheel, if one or both of the first actuators 548, 552 fail.

As shown in Figure 7A, the handle 540 includes a housing 700. The first actuator 548 extends into the housing 700 and is connected to a first rack 704 that is connected to a first gate 708. Similarly, the second actuator 552 extends into the housing 700 and is connected to a second rack 712 that is connected to a second gate 716. An energy storing device 720, such as a spring or piston, is connected to an implant sheath 800 and abuts the first gate 708. A first channel 724 is provided between the first gate 708 and the second gate 716 and a second channel 728 is provided between the second gate 716 and a stopper 732. A release catheter catch 734 is also provided adjacent the second gate 716, which is connected to the release catheter 450.

As shown in Figure 7B, the first actuator 548 is actuated in a downward motion (arrow 736), which pushes the first rack 704 to the left (arrow 740), pushing down gate 708 (arrow 744). By pushing down gate 708, the energy storing device 720 moves to the right (arrow 748), sliding within the first channel 724 until the energy storing device 720 abuts the second gate 716 and release catheter catch 734, as shown in Figure 7C. When the energy storing device 720 slides toward the second gate 716, the energy storing device 720 withdraws the outer sheath (not shown) of the catheter 544. As shown in Figure 7C, the second actuator 552 is actuated in a downward motion (arrow 752), which pushes the second rack 712 to the left (arrow 756), pushing up gate 716 (arrow 760). By pushing up gate 716, the energy storing device 720 along with the release catheter catch 734 moves to the right (arrow 764), sliding within the second channel 728 until the energy storing device 720 abuts the stopper 732, as shown in Figure 7D. When the energy storing device 720 slides toward the stopper 732, the energy storing device 720 further withdraws the implant sheath (not shown) along with the release catheter 450 (not shown).

Figure 7E illustrates an alternative handle configuration. In Figure 7E, the housing 700 includes a motor 768, a threaded shaft 772, a first sled 774, a second sled 778, a first rack 782 and a second rack 786. The motor 768 is coupled with the threaded shaft 772 so that the motor 768 rotates the threaded shaft 772 when it is powered on. The first sled 774 and second sled 778 are connected to the threaded shaft 768 at a first position and second position, respectively. The first sled 774 is coupled with the outer sheath of the delivery device 412 and the second sled 778 is coupled with the release catheter of the delivery device 412. The first rack 782 couples the first actuator 548 with the motor 768 and the second rack 786 couples the second actuator 552 with the motor 768.

As shown in Figure 7F, the first actuator 548 is actuated in a downward motion (arrow 790), deflecting the first rack 482, which powers the motor 768, thereby rotating the threaded shaft 772. Rotation of the threaded shaft 772 moves the first sled 774 toward the second sled 778, which remains stationary. The movement of the first sled 774 retracts the outer sheath of the catheter. When the first sled 774 contacts the second sled 778 (Figure 7G), power to the motor 768 is discontinued. As shown in Figure 7H, when the second actuator 552 is actuated in a downward motion (arrow 794), the second rack 786 is deflected, powering the motor 768 to again rotate the threaded shaft 772. Rotation of the threaded shaft 772 causes movement of the second sled 778 toward a proximal end of the housing 700 to retract the release catheter and outer sheath.

Figures 8A, 8B and 9 illustrate the distal end of the delivery device 412 and the implant. As shown in Figure 8A, the delivery system 412 includes an implant sheath 800 and a marker 802 disposed on the implant sheath 800. The marker 802 may be one or more of the various types of conventional markers such as an optically visible marker (e.g. a marker which is colored to distinguish from its surroundings) which is visible during a hysteroscopy by visible light and a camera or a radiopaque marker or an ultrasound marker (which is visible in an ultrasound image) or other known markers which allow the user of the delivery device 412 to guide and place the distal end of the system at a proper deployment position.

In Figure 8A, a distal portion 804 of the contraceptive device 820 is shown. The distal portion 804 includes a tip 808 and an attachment mechanism 812. The attachment mechanism 812 may be a solder bond. In certain embodiments, the distal portion 804 (such as the portion from the tip 808 to the attachment mechanism 812) may have a preformed bend of about 15° relative to the rest of the implant.

Figure 8B shows the delivery device 412 after the implant sheath 800 has been retracted (or alternatively, the contraceptive device has been pushed relative to the implant sheath 800) such that the contraceptive device 820 is fully viewable.

The contraceptive device 820 includes, in this embodiment, an outer coil 824 which is attached at attachment mechanism 812 to an inner coil 828 shown in Figure 9. The inner coil 828 may extend from the tip 808 in a proximal direction toward an end piece 832 which is attached to the outer coil 824. The outer coil 824 is thus coupled to the inner coil 828 at attachment 812 and coupled to the end piece 832 as shown in Figure 9. The end piece 832 may, in one embodiment, be coupled by a frictional fit to a release catheter or delivery wire such as the release catheter 450 shown in Fig 4A. In an alternative embodiment the end piece can be attached to, on one hand, to the inner coil (at a proximal portion of the inner coil) and on the other hand be releasably attached to the release catheter (and in this case the outer coil is not attached to the release catheter).

The contraceptive device 820 shown in Figures 8A-8B and 9 may also include a tissue in-growth agent 836. The tissue in-growth agent may be a polyester fiber or other types of agents designed to cause tissue in-growth to functionally occlude the fallopian tube. Functional occlusion of the fallopian tube is described, for example, in U.S. Patent 6,526,979 which is incorporated herein by reference.

Referring back to Figure 8B, the end piece 832 is adjacent to and abuts a release catheter 840. The release catheter 840 may include a pin or other interface designed to mate with a receptor or other interface on the end piece 832 to thereby couple the contraceptive device 820 to the release catheter 840. In one exemplary embodiment, the two interface elements on the release catheter 840 and the end piece 832 are coupled through an interference fit. The contraceptive device and the release catheter can be released by retracting the release catheter. In another exemplary embodiment, the two interface elements on the release catheter 840 and the end piece 832 may resemble a screw and a nut which more securely secures the contraceptive device and release catheter to each other. The contraceptive device and release catheter can be released by unscrewing the contraceptive device from the release catheter after the contraceptive device has been implanted.

The contraceptive device 820 shown in Figures 8A-8B and 9 resembles the Essure device from Conceptus, Inc. of Mountain View, California, in that there is an outer coil which may be formed from a superelastic or resilient member and an inner coil which is coupled to the outer coil. The outer coil is designed to radially expand to engage the walls of a portion of the fallopian tube to thereby engage those walls and hold the device within the fallopian tube.

It will be appreciated that other contraceptive devices and/or delivery device configurations may be used. For example, the contraceptive devices described in copending U.S. Application No. 10/866,493, filed June 10, 2004, entitled Medical Devices and Methods of Making and Using Such Medical Devices, the entirety of which is hereby incorporated by reference, may be used as an alternative to the contraceptive device 820 described above. Other exemplary contraceptive devices include spider-like, stent-like, coil-like, or other implantable contraceptive devices.

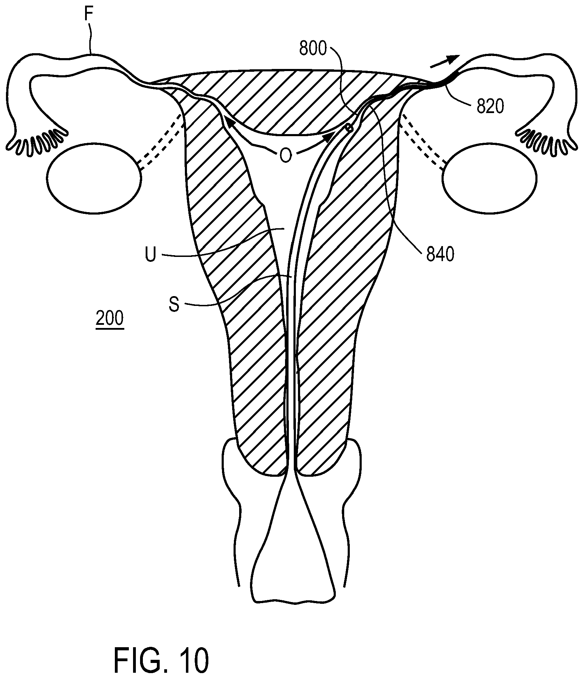

Figure 10 illustrates placement of the contraceptive device 820 in the ovarian pathway with the delivery sheath 200. As shown in Figure 10, the device 820 may extend proximally beyond ostium O into uterus U by a distance of, for example, about 0.2 to 1.2cm. The device 820 may also extend distally beyond the intermural section INT and/or uterotubal junction by a distance of, for example, at least about 0.6cm. It will be appreciated that the values provided above are exemplary and that the values may vary. In addition, it will be appreciated that there may be no proximal extension, no distal extension or neither proximal nor distal extension of the contraceptive device 820.

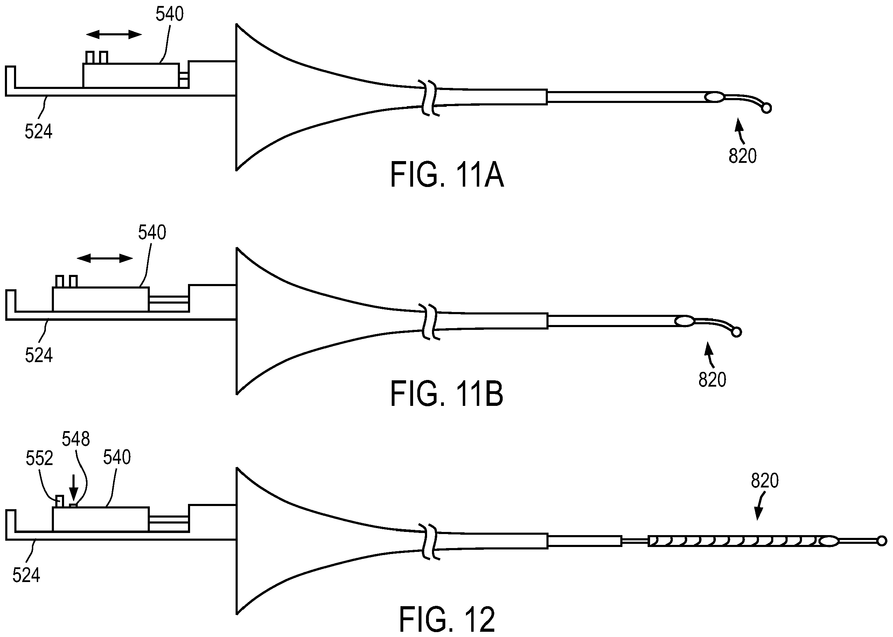

Referring to Figures 11A-13, delivery of the implant with the device delivery system 404 is illustrated in further detail. Referring to Figures 11A and 11B, the handle 540 of the delivery system 412 is slideable along the rail 524 of the guide 408. Sliding the handle 540 toward the sheath 200 pushes the contraceptive device 820 distally (Figure 11A), while sliding the handle 540 away from the sheath 200 causes the contraceptive device 820 to move proximally (Figure 11B). Thus, sliding the handle 540 along the rail 524 allows an operator to accurately position the contraceptive device at the target location and this can be done without moving the current position of the sheath 200 (which moving may disrupt the physician). As described above, the rail 524 may have a length between about 1cm and 11cm; thus, the operator can position the contraceptive device within about 1cm - 11cm from the initial delivery position. It will be appreciated that the value may less than 1cm or greater than 11cm.

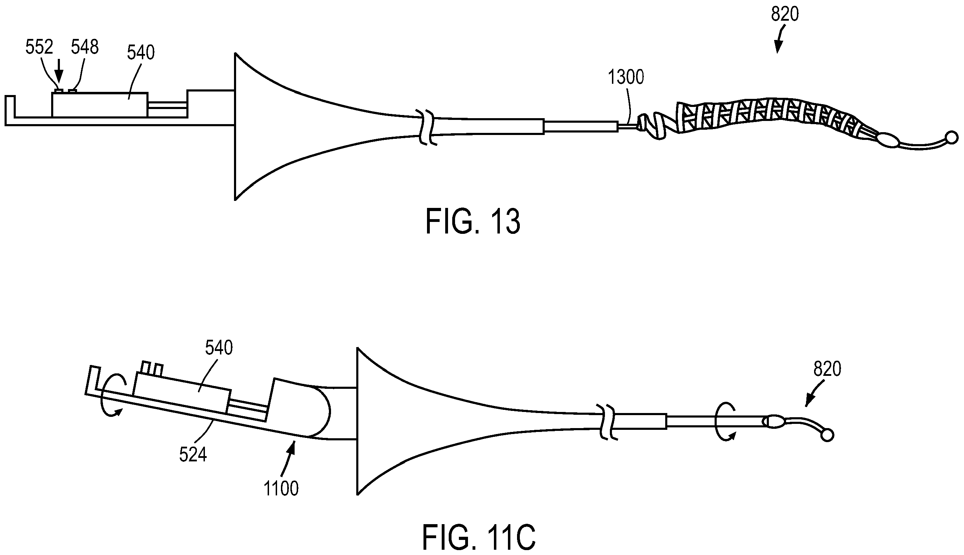

As shown in Figure 11C, the contraceptive device 820 is, optionally, rotatable. The ability to rotate the implant, without rotating the sheath 200, is particularly useful for those embodiments in which the implant has a preformed bend (or other non-linear conformation), such as a 15° bend (shown in Fig 11C). As shown in Figure 11C, the guide 408 includes a ball and socket 1100 that allows the rail 524 and handle 540 to rotate relative to the sheath 200. Rotational movement of the handle 540 results in rotational movement of the distal end of the delivery system 412, as shown in Figure 11C. The rotational movement can be used to position the contraceptive device in the fallopian tube or to separate the contraceptive device from the release catheter. It will be appreciated that although the ball and socket is shown as being a part of the guide 408, the ball and socket could alternatively be part of the handle, allowing rotational movement of the handle 540. In addition, it will be appreciated that although a ball and socket joint is described as allowing for rotational movement of the handle 540, it will be appreciated that alternative joints or devices may be located at the handle 540 or guide 408 to allow for rotational movement.

Figure 12 shows actuation of the first actuator 548, which results in exposure of the contraceptive device 820 via retraction of the implant sheath. It will be appreciated that, alternatively, actuation of the first actuator may result in exposure of the contraceptive device 820 by pushing the release catheter (e.g. release catheter 450) and, hence, the contraceptive device, distally from the delivery catheter.

As shown in Figure 13, actuation of the second actuator 552 results in expansion of the implant when the release catheter releases the implant. Actuation of the second actuator 552 may also retract the guidewire 1300 to release the contraceptive device 800 from the guide wire 1300.

It will be appreciated that in embodiments in which the contraceptive device is a self-expanding contraceptive device, actuation of the first actuator will result in both exposure and expansion of the contraceptive device 800 when the delivery catheter is retracted. In such an embodiment, actuation of the second actuator may simply result in release of the contraceptive device 800 from the release catheter. In alternative embodiments, the contraceptive device may be balloon-expandable; thus, actuation of the second actuator would expand and release the contraceptive device by expanding the balloon in such an embodiment.

In one embodiment, the delivery device includes an indicator (not shown). The indicator may indicate that the catheter has been retracted and then that the implant has been delivered. The indicator may be visual or audible.

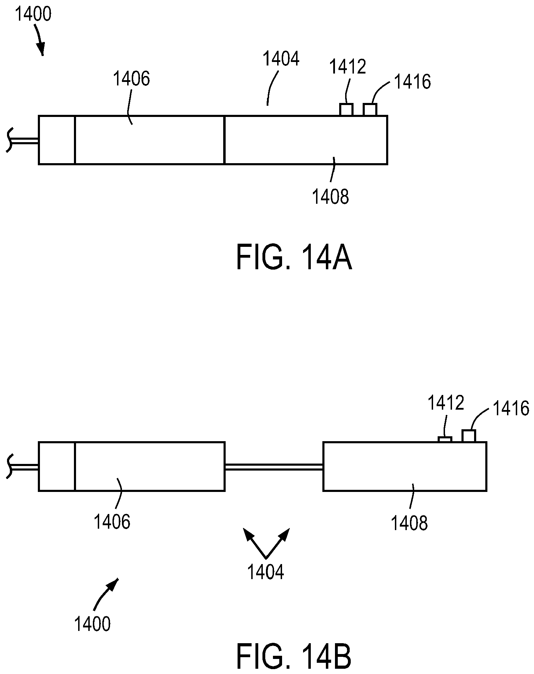

Figures 14A and 14B illustrate an alternative delivery device configuration. The delivery device 1400 includes a handle 1404 having a first handle portion 1406 and a second handle portion 1408. A first actuator 1412 and a second actuator 1416 are provided on the handle 1400. Although the first and second actuators 1412, 1416 are shown on the second handle portion 1408, it will be appreciated that the actuators 1412, 1416 can alternatively be placed on the first handle portion 1406, or the first actuator 1412 can be placed on the first handle portion 1406 and the second actuator 1416 placed on the second handle portion 1408. A delivery catheter 1420 is also shown extending from the handle 1404.

As shown in Figure 14B, actuation of the first actuator 1412 causes separation of the first handle portion 1406 and the second handle portion 1408. The second handle portion 1408 moves distally relative to the first handle portion 1406 resulting in retraction of the delivery catheter to expose a contraceptive device, as described above. Actuation of the second actuator 1416 causes expansion of the contraceptive device and release of the contraceptive device from the release catheter, as described above.

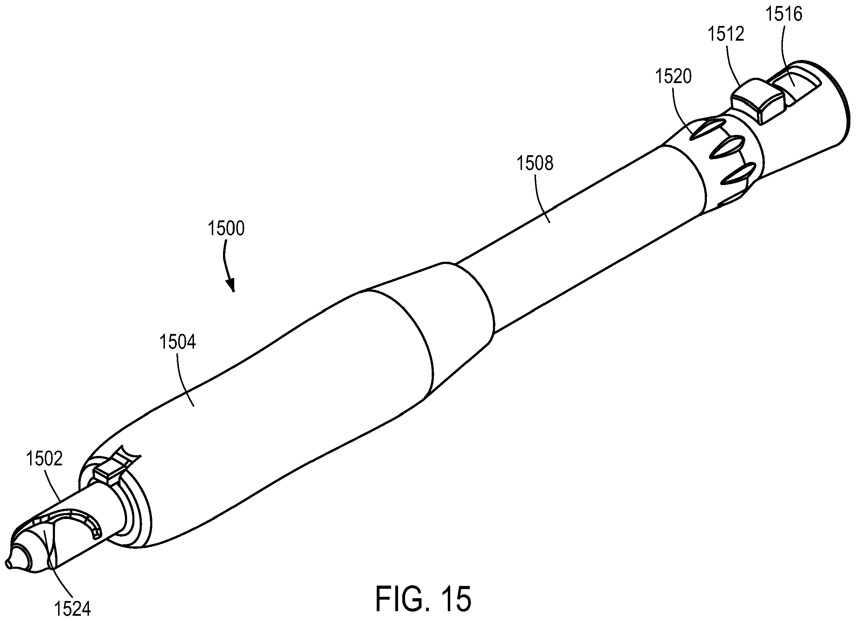

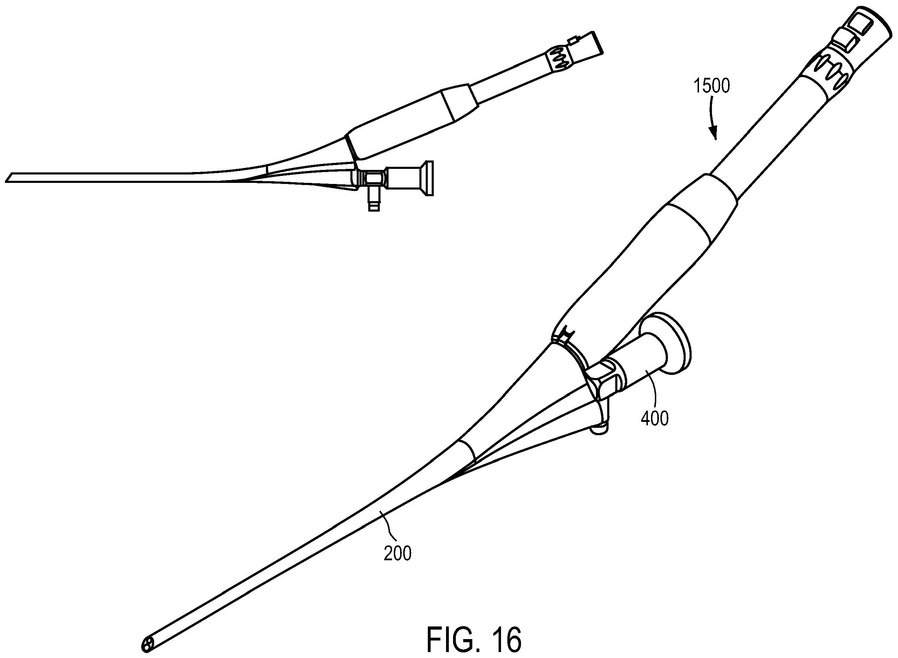

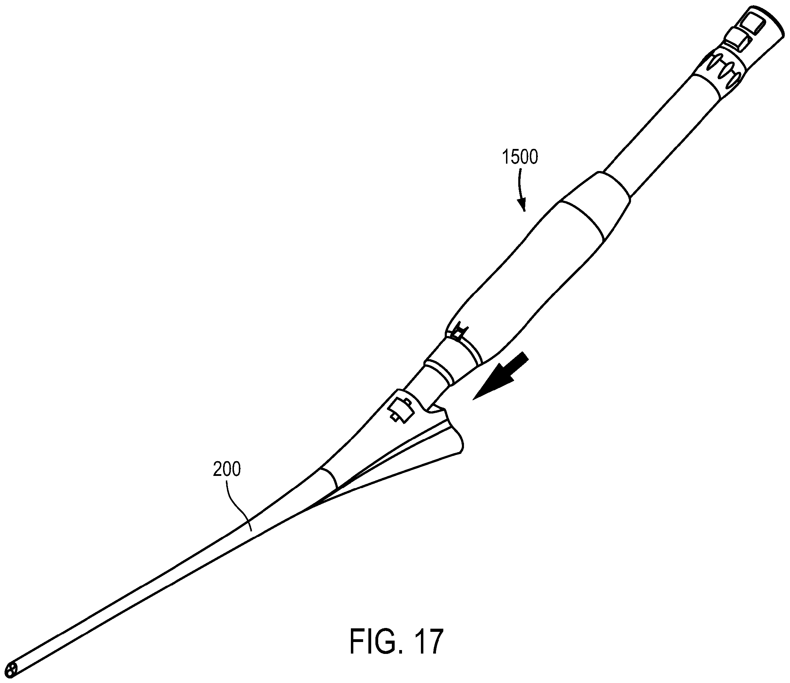

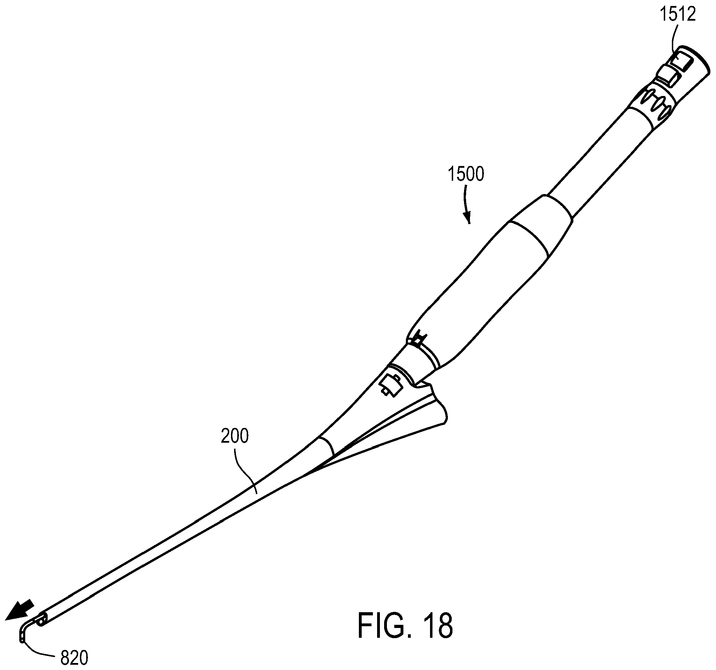

Figure 15 illustrates an alternative delivery system 1500 for delivering a contraceptive device at a fallopian tube of a patient. The delivery system 1500 includes an interlocking element 1502, a main body 1504, a secondary body 1508, a first actuator 1512 and a second actuator 1516. The delivery system 1500 may also include a knob 1520. The interlocking element 1502 is similar to the interlocking element of the guide 408, and is configured to be inserted into the second lumen 212 of the sheath 200, and includes a groove 1524 that engages with the tab in the second lumen 212 of the sheath 200 to lock the delivery system 1500 to the sheath 200, as shown in Figure 16. Figure 17 illustrates connection of the delivery system 1500 to the sheath 200 in further detail. Actuation of the actuators 1512 and 1516 operate in a manner similar to the actuators of the delivery device 412 to deliver the contraceptive device in the fallopian tube, as shown in Figure 18.

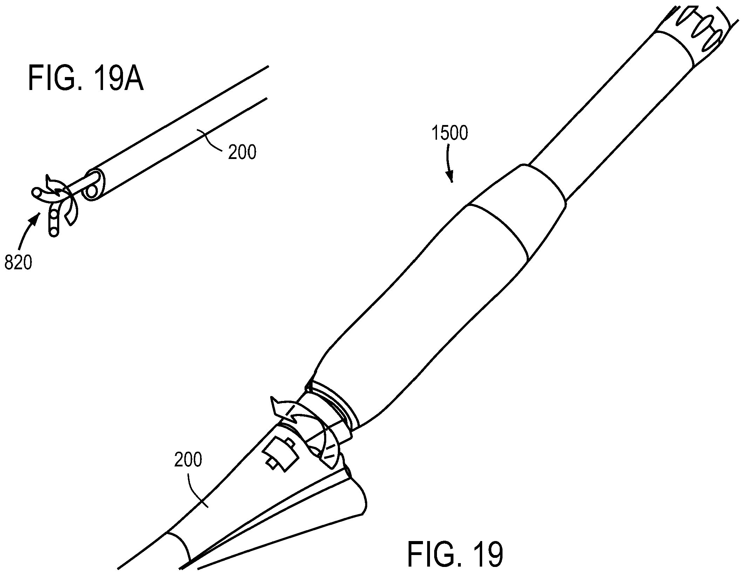

Figures 19 and 19A illustrate an alternative embodiment of the sheath 200 that includes a rotational element 1900 that allows for rotation of the delivery system 1500 and thus the contraceptive device.

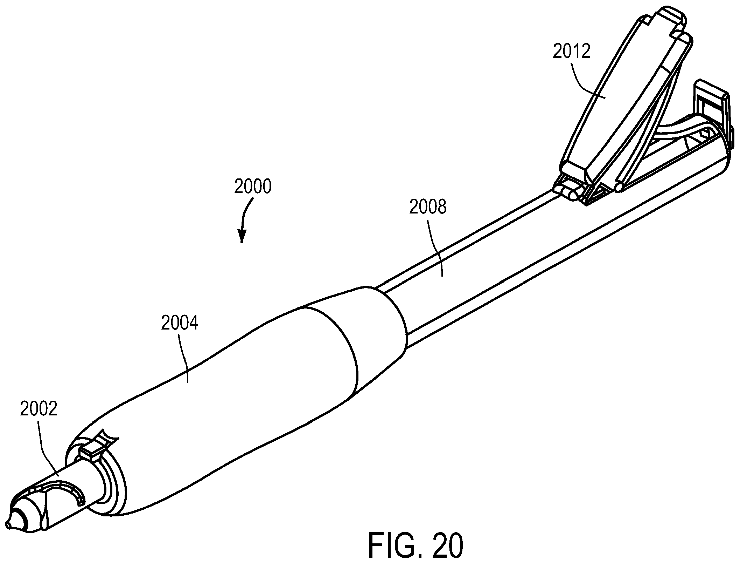

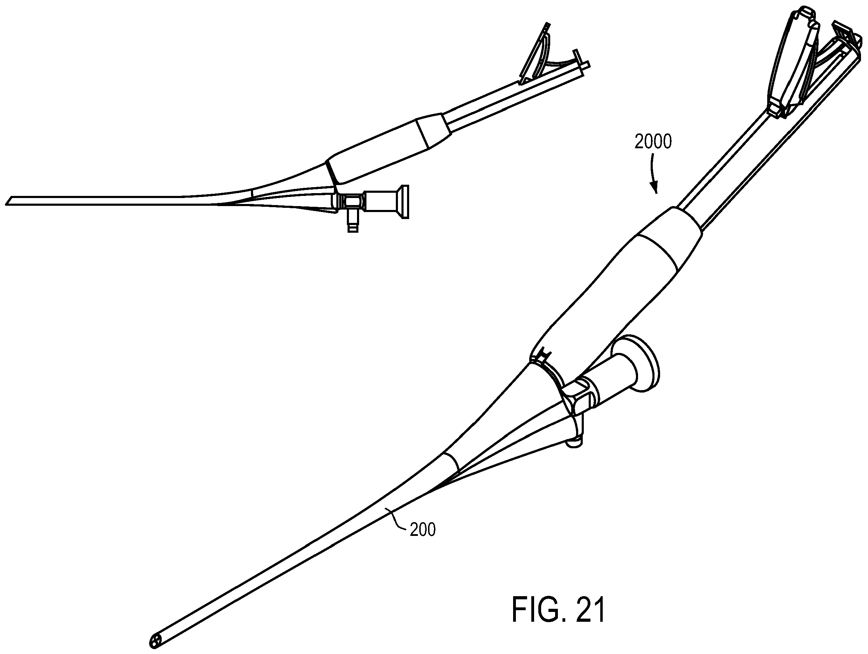

Figure 20 illustrates an alternative delivery system 2000 for delivering a contraceptive device at a fallopian tube of a patient. The delivery system 2000 includes an interlocking element 2002, a main body 2004, a secondary body 2008 and an actuator 2012. The actuator 2012 has a clamshell design that includes a releasable lock 2016 and a clamshell 2020 that is moveable in an opening 2024 in the secondary body 2008. Figure 21 illustrates connection of the delivery system 2000 to the sheath 200. The delivery system 2000 is connected to the sheath 200 as described above with reference to Figures 15-17.

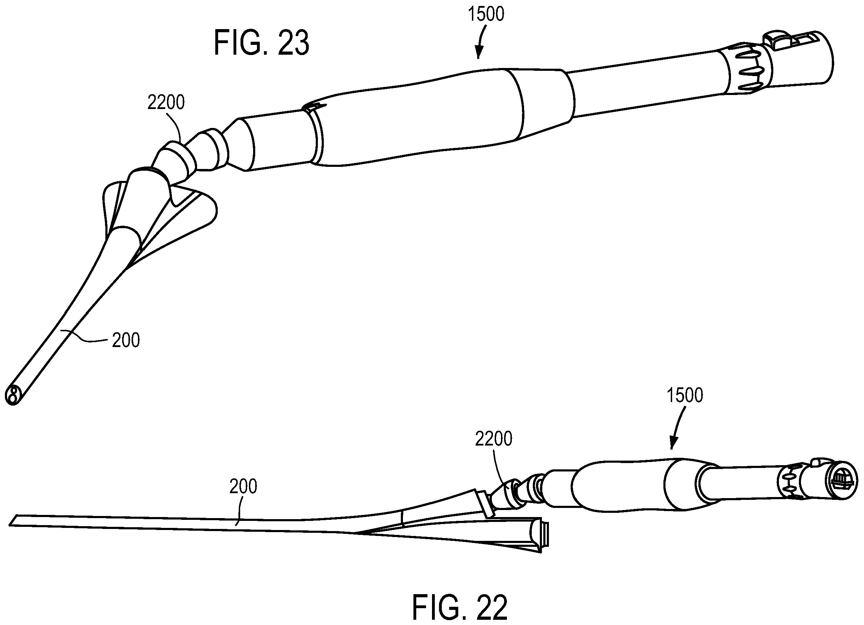

Figures 22 and 23 illustrate a flexibility extension 2200 that may be provided between the sheath and a delivery system, such as the delivery system shown in Figure 15. The flexibility extension 2200 allows the delivery system to be positioned away from the proximal end of the sheath. It will be appreciated that the flexibility extension 2200 can be used with any of the other delivery systems described herein.

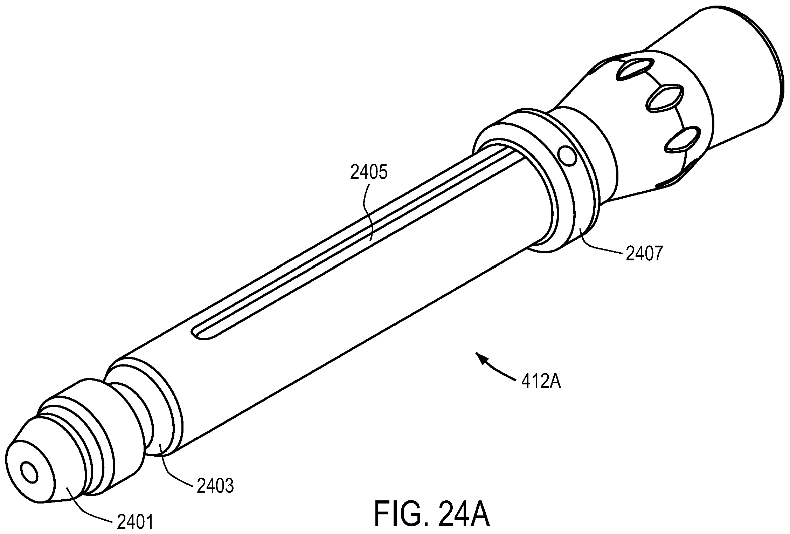

Figures 24A, 24B, 25A, 25B, 25C, 26 and 27 show another embodiment of a delivery device according to the present invention. In this embodiment, rather than having a handle which moves on a rail on a guide, the handle has a control (e.g. a longitudinal placement control) which moves on the handle to position, longitudinally (in a proximal/distal direction), the implant without requiring movement of the handle longitudinally and without requiring movement of the hysteroscope. In one implementation, the control may be a ring, coupled through a slot in the handle to the release catheter or delivery wire, and this ring is moved along the exterior of the handle to in turn move the implant longitudinally; moreover, the entire handle may be rotated to rotate the implant for proper delivery, and the handle may be swiveled to a side of the working channel.

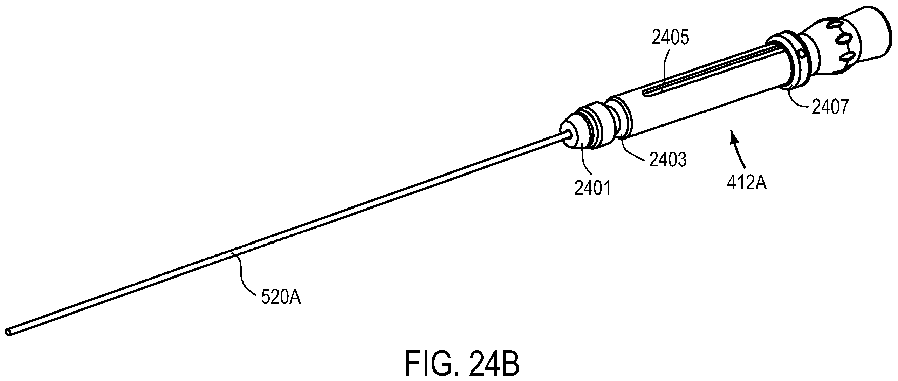

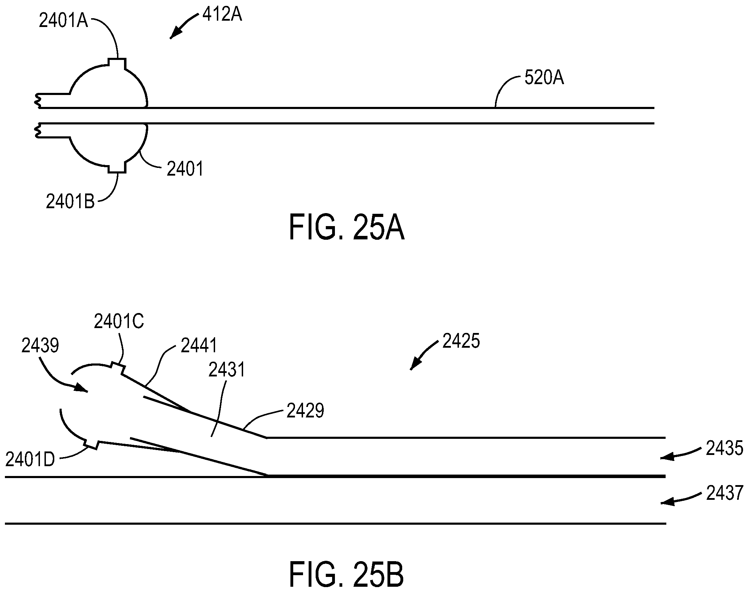

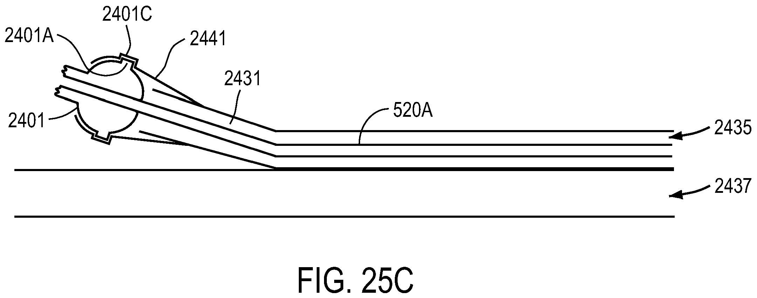

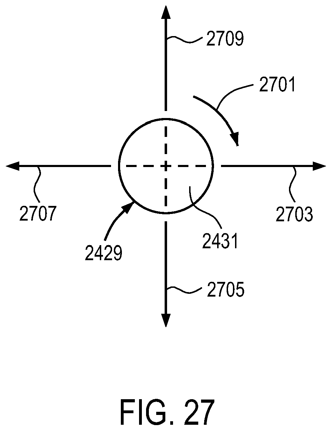

The delivery device 412A shown in the perspective view of Figure 24A includes a handle having a slot 2405 and an interlocking end piece 2401. The interlocking end piece 2401 is designed to be attached to a coupler or adapter on a hysteroscope sheath, such as the coupler 2441 shown in FIGS 25B and 25C; the interlocking end piece 2401 may be removably attached to the coupler or adapter through an interaction between structures on the end piece (e.g. a groove, a ridge, etc.) and corresponding structures on the coupler or adapter (e.g. a ridge, a groove, etc.). The slot 2405 is designed to allow longitudinal movement of the longitudinal placement control 2407 along the length of the handle; moving the control distally moves the implant distally relative to the handle and moving the control proximally moves the implant proximally relative to the handle. In other implementations, other types of controls may be used such as a rotating wheel or a button coupled to a spring or electric motor designed to move the implant longitudinally. The delivery device 412A may optionally include a ball joint 2403; in the embodiment shown in Figure 24A and Figure 24B, the ball joint connects the end piece 2401 with the rest of the delivery device 412A and allows the handle of the delivery device to be rotated and swiveled (see Figure 27) relative to the working channel and the hysteroscope sheath.

The delivery device 412A is shown in Figure 24B with a protective sheath 520A attached to the distal portion of the interlocking end piece 2401. This protective sheath 520A may be similar to protective sheath 520 described above; for example, it may be relatively hard (e.g. a metal) and may protect the release catheter and the implant from harm when the deliver device 412A is inserted into a working channel of a hysteroscope sheath and it may be less flexible than the release catheter and any implant sheath which protects and surrounds at least a portion of the implant.

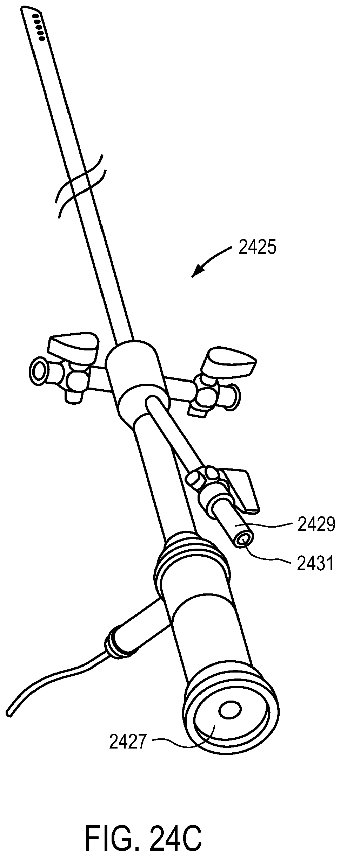

Figure 24C shows an example of a conventional hysteroscope sheath 2425 which may be used with one or more embodiments of the delivery devices described herein. The hysteroscope sheath 2425 may have a plurality of channels just as the delivery sheath 200 can have a plurality of channels. For example, the hysteroscope sheath 2425 may have a visualization channel (which includes a hysteroscope lumen 2437) configured to receive a hysteroscope, such as hysteroscope 2427, and a working channel 2429, configured to receive any one of the delivery devices described herein such as the delivery device 412A, and other channels such as fluid inlet and fluid outlet channels. The working channel 2429 includes a lumen 2431 configured to receive any one of the delivery devices described herein. The hysteroscope sheath shown in Figure 24C is a rigid sheath and may be used with a rigid hysteroscope; in other embodiments, a flexible hysteroscope and/or a flexible hysteroscope sheath may be used with a delivery device described herein.

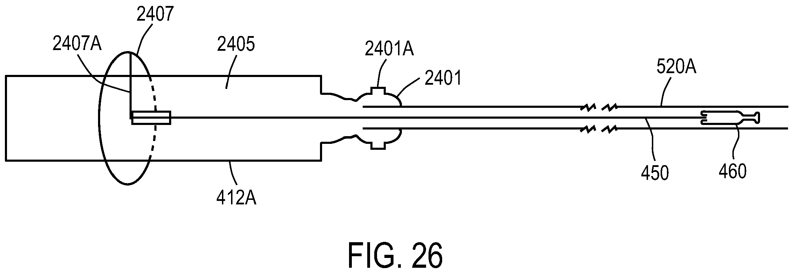

The delivery device 412A includes an interlocking end piece 2401 which is configured to be attached removably to a hysteroscope sheath through cooperating structures on the end piece and on a portion of a couple of the hysteroscope sheath or an adapter fitted on or coupled to the hysteroscope sheath. Figure 25A shows a cross sectional view of a distal portion of the delivery device 412 A. This view shows that the interlocking end piece 2401 includes two ridges 2401A and 2401B, and these ridges may be separate (to fixedly lock the end piece to the coupler) or may run along the entire perimeter of the end piece as a continuous ridge (to allow rotation of the end piece within the couple or adapter 2441). The end piece has lumen through it and extending into the protective sheath, and the release catheter and the implant sheath (if any) and the implant are configured to be disposed in that lumen such that the implant can be initially within the protective sheath (see Figure 26) and then move distally out of the protective sheath to be delivered.

If the ridges 2401A and 2401B are separate they may be used to fit into and lock the end piece into the coupler 2441 to prevent the end piece from rotating within the coupler 2441. On the other hand, if the ridges run the entire perimeter of the end piece, thereby forming a continuous ridge, then a corresponding continuous groove which mates with that ridge will allow the end piece to rotate within the coupler 2441. In those implementations in which the end piece is non-rotatably fixed within the coupler 2441, rotation of the handle relative to the hysteroscope sheath may be provided by the optional ball joint 2403 shown in Figure 24A.

Figure 25B shows a cross sectional view of the hysteroscope sheath 2425 which includes two lumens, which are the working lumen 2431 and the hysteroscope lumen 2437. The working lumen 2431 ends at a distal end 2534 of the lumen of the working channel. The working channel 2429 includes a coupler 2441 or adapter 2441 which is used to attach the delivery device 412A to the working channel. The coupler 2441 includes the socket 2439 which is designed to receive the end piece 2401, and this socket includes two grooves 2401C and 2401D. In one embodiment, these grooves may be separate and distinct and be designed to mate with separate and distinct ridges 2401A and 2401B, respectively, in order to fixably lock the end piece relative to the coupler, thereby preventing rotation (but rotation can still be allowed with the optional ball joint 2403). In other implementations, the groove may be continuous around the inner perimeter of the socket 2439 thereby allowing the ridges 2401A and 2401B to move within that groove and to allow rotational movement of the end piece 2401 relative to the coupler 2441. The coupler 2441 may be permanently secured to the hysteroscope sheath 2425 or may be removeably coupled to the working channel 2429 of the hysteroscope sheath 2425. The coupler or adapter may fit into the working channel or fit on the outside of the working channel (as shown in Figure 25B which shows a fit on the outside). The coupler or adapter may be secured by glue, a clamp, tongue and groove interface, etc. In certain embodiments, the adapter or coupler may be built into the hysteroscope sheath. While Figure 25B shows that the working channel lumen and the hysteroscope lumen are separate channels, in other embodiments, the channels may be merged into one channel or lumen along at least a portion of the hysteroscope sheath 2425. In alternative embodiments, the delivery device 412A may be used with other types of hysteroscope sheaths, such as the hysteroscope sheaths shown in Figures 2 and 3.

Figure 25C shows a cross-sectional view of the distal portion of a delivery device 412A shown inserted into the working channel of the hysteroscope sheath 2425. In particular, the protective sheath 520A has been inserted into the lumen 2431 of the working channel. Even though the protective sheath may be hard (e.g., formed of a metal) it can still bend in order to place the sheath into the lumen 2431 of the working channel. Figure 25C also shows that end piece 2401 has been secured into the coupler 2441 by having the ridges on the end piece mate with corresponding grooves, or a single groove in the coupler 2441. The release catheter 450 and the implant 460 are not shown in the view of Figure 25C in order to simplify that view, but it will be understood that they are present in the typical embodiment of the present invention.

Figure 26 shows a cross sectional view of the delivery device 412A. The protective sheath 520A is shown protecting the implant 460 within the protective sheath, and the implant 460 is coupled to the release catheter or delivery wire 450 in order to allow longitudinal placement of the implant 460 through longitudinal manipulation of the longitudinal placement control 2407 which may be a ring with a post 2407A extending through the slot 2405 and coupled to a distal end of the release catheter 450. Distal movement of the control 2407 moves the implant 460 distally relative to the delivery device 412A while proximal movement of the control 2407 retracts the implant 460 back into the protective sheath 520A. In a typical delivery sequence, the implant 460 is retracted proximally so that it is protected while the protected sheath 520A is inserted into the lumen 2431 of the working channel of the hysteroscope sheath 2425. After the delivery device 412A has been properly positioned relative to the coupler 2441 as shown in Figure 25C, the implant can be extended out distally out past the protective sheath 520A and positioned properly relative to the fallopian tube to deploy the implant 460 at the conventional and known positions for deploying such implants within a fallopian tube. It will be understood that actuators, such as the first and second actuators may be included on the delivery device 412A to allow retraction of the implant sheath as described above and to allow release of the release catheter 450 from the implant 460 after the implant 460 has been deployed in the proper location of a fallopian tube. For example, a first actuator can include a stretched spring which stores a force which is used, when the stretched spring is allowed to relax, to retract the implant sheath; one end of the stretched spring can be coupled to a proximal portion of a handle of the delivery device 412A and the other end of the stretched spring can be coupled to the implant sheath and to a button or other user control. When the user presses the button (or activates the other user control) the button (or other user control) can release a stop on the spring to allow the spring to relax and thereby pull back the implant sheath. The button (or other user control) can itself form a stop by fitting through an opening in the handle and in doing so, prevent the spring from relaxing; when the button is depressed, it can be released from the opening and then allow the spring to relax. The release of the implant 460 from the release catheter 450 can occur concurrently with the retraction of the implant sheath or separately. In one implementation which uses a concurrent release, a single actuator can pull back the sheath and release the implant 460 from the release catheter (and there would be no second actuator in this implementation). This implementation can use, for example, a necked down (reduced diameter) sheath along a portion of the sheath to grasp and pull back the release catheter 450 from the implant 460 to thereby release the implant 460 from the release catheter 450. In another implementation which uses a separate release, a second actuator can be activated to cause release of the implant 460 from the release catheter 450, and this second actuator can also use a stretched spring which stores a force which is used, when the spring is allowed to relax, to pull the release catheter 450 from the implant 460.

Figure 27 shows how, in at least certain embodiments, the delivery device 412A may be rotated and swiveled relative to the working channel 2429 of the hysteroscope. The view of Figure 27 is looking down the lumen 2431 of the working channel 2429. Arrow 2701 represents pure rotational movement around the central axis of the lumen 2431. This pure rotational movement may occur by rotating the handle of the delivery device 412A without swiveling from left to right or up and down the handle relative to the working channel. This rotational capability may be provide by a rotational coupling between the end piece 2401 and the adapter 2441. In other embodiments, this rotational movement may be provided by the optional ball joint 2403. In addition, in those embodiments which use an optional ball joint 2403, the handle may be swiveled relative to the working channel by moving either left to right or up and down or other directions or combinations of swiveling and rotation. Arrows 2707 and 2703 show a left or right swivel, respectively, while arrows 2705 and 2709 show an up and down swivel, respectively. It will be understood that these swivels may occur at any angle rather than at zero, 90 degrees, 180 degrees, and 270 degrees as shown in Figure 27. In addition, the handle may be swiveled at any angle while at the same time being able to rotate the handle as shown by rotational movement 2701, particularly in to those embodiments which employ a ball joint 2403.

## Claims

1. A system for delivering an implant to a fallopian tube of a female body comprising:
a delivery sheath (200) having a proximal end and a distal end,
a delivery device (412) that acts as a handle, the delivery device having a proximal end and a distal end (412b), the delivery device comprising a ball (466) located near the distal end (412b) of the delivery device;
an implant sheath (800) having a proximal end and a distal end, the proximal end of the implant sheath connected to the delivery device (412);
a release catheter (450, 850) having a proximal end and a distal end, the proximal end of the release catheter (450, 850) connected to the delivery device (412);
an implant releasably coupled with the distal end of the release catheter; and
a guide (408) comprising:
a body;
a rail (524) extending proximally from the body, wherein the delivery device (412) is slideably engaged with the rail (524);
a locking mechanism (516) to lock the guide to the a delivery sheath (200); and
a protective sheath (520) extending distally from the body, the release catheter (450, 850), the implant sheath (800), and the implant slideable through the protective sheath (520);
wherein the delivery device (412) is coupled to the rail (524) of the guide (408) by a tab (462) which rides in the rail (524) to allow longitudinal movement (468) of the delivery device (412),
wherein the tab (462) is coupled to a socket joint (464) for receiving and holding the ball (466),
wherein the ball (466) and the socket joint (464) are rotatably coupled, and
wherein the ball (466) forms a lumen to allow the implant sheath (800) and the release catheter (450, 850) to pass through the ball (466).

2. The implant delivery system of claim 1, wherein the implant comprises an expandable tubular member (824) and a tissue in-growth promoting agent (836)and wherein the implant can be moved longitudinally, relative to the guide (408), by moving the handle along the guide.

3. The implant delivery system of claim 2, wherein the handle comprises a first actuator (548) and a second actuator (552).

4. The implant delivery system of claim 3, wherein the first actuator (548) is configured to retract proximally the implant sheath (800), to expose the implant and wherein the second actuator (552) is configured to release the implant from the release catheter.

5. The implant delivery system of claim 4, wherein the implant sheath (800) and the release catheter (450) are more flexible than the protective sheath (520).

6. The implant delivery system of claim 5, wherein the protective sheath (520) is metallic and wherein the implant sheath (800) and the release catheter (450) comprise a flexible polymer.

7. The implant delivery system of claim 2, wherein the handle comprises a first actuator (548) and a second actuator (552) and wherein the first actuator is configured to extend the implant distally away from the handle, and wherein the second actuator is configured to release the implant from the release catheter.

8. The implant delivery system of claim 1, wherein the delivery sheath (200) includes a port (208) to receive a visualization system.

9. The implant delivery system of claim 1, wherein the guide (408) restrains the longitudinal movement of the handle.

10. The implant delivery system of claim 1 wherein the rail (524) comprises a stopper (528) on the rail, and wherein the handle is slideable on the rail between the body and the stopper.

## Patentansprüche

1. System zum Zuführen eines Implantats zu einem Eileiter eines weiblichen Körpers, umfassend:
eine Zuführhülse (200) mit einem proximalen Ende und einem distalen Ende,
eine Zuführvorrichtung (412), die als ein Griff wirkt, wobei die Zuführvorrichtung ein proximales Ende und ein distales Ende (412b) hat, wobei die Zuführvorrichtung einen Kugelkopf (466) umfasst, der in der Nähe des distalen Endes (412b) der Zuführvorrichtung angeordnet ist,
eine Implantathülse (800) mit einem proximalen Ende und einem distalen Ende, wobei das proximale Ende der Implantathülse mit der Zuführvorrichtung (412) verbunden ist,
einen Freigabekatheter (450, 850) mit einem proximalen Ende und einem distalen Ende, wobei das proximale Ende des Freigabekatheters (450, 850) mit der Zuführvorrichtung (412) verbunden ist,
ein Implantat, das freigebbar mit dem distalen Ende des Freigabekatheters gekoppelt ist, und
eine Führung (408), die Folgendes umfasst:
einen Körper,
eine Schiene (524), die sich proximal vom Körper erstreckt, wobei die Zuführvorrichtung (412) mit der Schiene (524) in Verschiebeeingriff steht,
einen Verriegelungsmechanismus (516), um die Führung an eine Zuführhülse (200) zu verriegeln, und
eine Schutzhülse (520), die sich distal vom Körper erstreckt, wobei der Freigabekatheter (450, 850), die Implantathülse (800) und das Implantat durch die Schutzhülse (520) geschoben werden können,
wobei die Zuführvorrichtung (412) durch eine Lasche (462) an die Schiene (524) der Führung (408) gekoppelt ist, wobei die Lasche (462) in der Schiene (524) fährt, um eine Längsbewegung (468) der Zuführvorrichtung (412) zu gestatten,
wobei die Lasche (462) an eine Gelenkpfanne (464) zum Aufnehmen und Halten des Kugelkopfs (466) gekoppelt ist,
wobei der Kugelkopf (466) und die Gelenkpfanne (464) drehbar gekoppelt sind und
wobei der Kugelkopf (466) ein Lumen bildet, damit die Implantathülse (800) und der Freigabekatheter (450, 850) durch den Kugelkopf (466) gehen können.

2. Implantatzuführsystem nach Anspruch 1, wobei das Implantat ein expandierbares röhrenförmiges Glied (824) und ein Gewebeeinwachsfördermittel (836) umfasst und wobei das Implantat durch Bewegen des Griffs entlang der Führung in Längsrichtung bezüglich der Führung (408) bewegt werden kann.

3. Implantatzuführsystem nach Anspruch 2, wobei der Griff einen ersten Aktuator (548) und einen zweiten Aktuator (552) umfasst.

4. Implantatzuführsystem nach Anspruch 3, wobei der erste Aktuator (548) dazu konfiguriert ist, die Implantathülse (800) proximal zurückzuziehen, um das Implantat freizulegen, und wobei der zweite Aktuator (552) dazu konfiguriert ist, das Implantat vom Freigabekatheter freizugeben.

5. Implantatzuführsystem nach Anspruch 4, wobei die Implantathülse (800) und der Freigabekatheter (450) flexibler als die Schutzhülse (520) sind.

6. Implantatzuführsystem nach Anspruch 5, wobei die Schutzhülse (520) metallisch ist und wobei die Implantathülse (800) und der Freigabekatheter (450) ein flexibles Polymer umfassen.

7. Implantatzuführsystem nach Anspruch 2, wobei der Griff einen ersten Aktuator (548) und einen zweiten Aktuator (552) umfasst und wobei der erste Aktuator dazu konfiguriert ist, das Implantat vom Griff weg distal auszufahren, und wobei der zweite Aktuator dazu konfiguriert ist, das Implantat vom Freigabekatheter freizugeben.

8. Implantatzuführsystem nach Anspruch 1, wobei die Zuführhülse (200) einen Port (208) zur Aufnahme eines Visualisierungssystems aufweist.

9. Implantatzuführsystem nach Anspruch 1, wobei die Führung (408) die Längsbewegung des Griffs hemmt.

10. Implantatzuführsystem nach Anspruch 1, wobei die Schiene (524) einen Pfropfen (528) auf der Schiene umfasst und wobei der Griff auf der Schiene zwischen dem Körper und dem Pfropfen verschiebbar ist.

## Revendications

1. Système pour introduire un implant dans une trompe de Fallope dans le corps d'une femme, comprenant :
une gaine d'introduction (200) ayant une extrémité proximale et une extrémité distale,
un dispositif d'introduction (412) servant de poignée, le dispositif d'introduction ayant une extrémité proximale et une extrémité distale (412b), le dispositif d'introduction comprenant une bille (466) située à proximité de l'extrémité distale (412b) du dispositif d'introduction ;
une gaine d'implant (800) ayant une extrémité proximale et une extrémité distale, l'extrémité proximale de la gaine d'implant étant connectée au dispositif d'introduction (412) ;
un cathéter de libération (450, 850) ayant une extrémité proximale et une extrémité distale,
l'extrémité proximale du cathéter de libération (450, 850) étant connectée au dispositif d'introduction (412) ;
un implant accouplé de manière libérable à l'extrémité distale du cathéter de libération ; et
un guide (408) comprenant :
un corps ;
un rail (524) s'étendant dans la direction proximale depuis le corps, le dispositif d'introduction (412) pouvant être engagé par coulissement avec le rail (524) ;
un mécanisme de verrouillage (516) pour verrouiller le guide à la gaine d'introduction (200) ; et
une gaine de protection (520) s'étendant dans la direction distale depuis le corps, le cathéter de libération (450, 150), la gaine d'implant (800) et l'implant pouvant coulisser à travers la gaine de protection (520) ;
le dispositif d'introduction (412) étant accouplé au rail (524) du guide (408) par une languette (462) qui se déplace dans le rail (524) pour permettre un mouvement longitudinal (468) du dispositif d'introduction (412),
la languette (462) étant accouplée à un joint à rotule (464) pour recevoir et retenir la bille (466),
la bille (466) et le joint à rotule (464) étant accouplés de manière rotative, et
la bille (466) formant une lumière pour permettre à la gaine d'implant (800) et au cathéter de libération (450, 850) de passer à travers la bille (466) .

2. Système d'introduction d'implant selon la revendication 1, dans lequel l'implant comprend un organe tubulaire expansible (824) et un agent promoteur de croissance tissulaire (836) et dans lequel l'implant peut être déplacé longitudinalement par rapport au guide (408) en déplaçant la poignée le long du guide.

3. Système d'introduction d'implant selon la revendication 2, dans lequel la poignée comprend un premier actionneur (548) et un deuxième actionneur (552).

4. Système d'introduction d'implant selon la revendication 3, dans lequel le premier actionneur (548) est configuré pour rétracter en direction proximale la gaine d'implant (800) pour exposer l'implant et dans lequel le deuxième actionneur (552) est configuré pour libérer l'implant du cathéter de libération.

5. Système d'introduction d'implant selon la revendication 4, dans lequel la gaine d'implant (800) et le cathéter de libération (450) sont plus flexibles que la gaine de protection (520).

6. Système d'introduction d'implant selon la revendication 5, dans lequel la gaine de protection (520) est métallique et dans lequel la gaine d'implant (800) et le cathéter de libération (450) comprennent un polymère flexible.

7. Système d'introduction d'implant selon la revendication 2, dans lequel la poignée comprend un premier actionneur (548) et un deuxième actionneur (552) et dans lequel le premier actionneur est configuré pour prolonger l'implant en direction distale à l'écart de la poignée, et dans lequel le deuxième actionneur est configuré pour libérer l'implant du cathéter de libération.

8. Système d'introduction d'implant selon la revendication 1, dans lequel la gaine d'introduction (200) comporte un orifice (208) pour recevoir un système de visualisation.

9. Système d'introduction d'implant selon la revendication 1, dans lequel le guide (408) limite le mouvement longitudinal de la poignée.

10. Système d'introduction d'implant selon la revendication 1, dans lequel le rail (524) comprend une butée (528) sur le rail, et dans lequel la poignée peut glisser sur le rail entre le corps et la butée.
